# EUROPEAN PATENT APPLICATION

(11) **EP 1 277 478 A1**
(43) Date of publication of application: **22.01.2003**
(21) Application number: 01925892.0
(22) Date of filing: 25.04.2001
(51) Int. Cl.: A61K 45/00, A61K 31/52, A61P 25/28, G01N 33/15, G01N 33/50

(54) **MEDICINAL COMPOSITIONS FOR SUPPRESSING BETA-AMYLOID PRODUCTION**

(30) Priority: 28.04.2000 JP 2000131037
(71) Applicant: YAMANOUCHI PHARMACEUTICAL CO. LTD., Tokyo 103-8411 (JP); Suzuki, Toshiharu, Chiba-shi, Chiba 263-0022 (JP)
(72) Inventor: SUZUKI, Toshiharu, 4-101, Todainishichibashukusha, Chiba-shi, Chiba 263-02212 (JP); WATANABE, Toru, Tsukuba-shi, Ibaraki 305-8585 (JP); KAWABATA, Shigeki, Tsukuba-shi, Ibaraki 305-8585 (JP); HACHIYA, Shunichiro, Tsukuba-shi, Ibaraki 305-8585 (JP)
(74) Representative: HOFFMANN - EITLE
(86) International application number: JP0103555
(87) International publication number: WO01082967

(57) **Abstract**

To provide pharmaceutical compositions for suppressing β-amyloid production on the basis of the cyclin-dependent kinase inhibitory activity. When a substance having a cyclin-dependent kinase inhibitory activity was allowed to contact with nerve cells, the β-amyloid production was suppressed.

## Description

### Technical Field

This invention relates to medicaments, particularly a pharmaceutical composition for suppressing β-amyloid (Aβ) production which contains a substance having the cyclin-dependent kinase (Cdk) inhibitory activity as the active ingredient, a method for detecting Aβ production by allowing the substance having Cdk inhibitory activity to contact with cells, and a method for treating dementia or Alzheimer's disease (AD) which comprises administering the pharmaceutical composition for suppressing Aβ production.

### Background of the Invention

Dementia is becoming a serious problem in an aging society. Dementia is a condition under which daily life and social life cannot be sufficiently conducted in various mental functions (e.g., memory, judgement, comprehension, language, capacity, cognition, feeling, volition, character and the like) due to cerebral organic disorders. Among them, AD is characterized by pathological changes such as falling off of nerve cells, reduction of synapse numbers, senile plaque in the brain, accumulation of neurofibrillary change and the like (*Science* (1990) 248, 1058 - 1060, *N. Engl*. *J. Med.* (1986) 314, 964 - 973 and *Neurobiol. Aging* (1995) 16, 365 - 380). The amyloid substance of senile plaque is a protein called Aβ which is formed when amyloid precursor protein (APP) undergoes metabolism (*Nature* (1987) 325, 733 - 736). Two metabolic pathways are known regarding the metabolism of APP, namely a pathway in which it undergoes cutting by α-secretase at the center of the Aβ domain (amyloid non-producing pathway) and a pathway in which it produces Aβ by undergoing Aβ domain preservative cutting by β-secretase and further undergoing cutting by γ-secretase (amyloid producing pathway) (Racchi, M. (1999) *Trends Pharmacol. Sci.* 20, 418 - 423, Sinha, S. (1999) *proc. Natl. Acad. Sci. USA* 96, 11049 - 11053). It is known that the ratio of APP metabolized by these two pathways, namely the amount of Aβ production, is changed by the influence of an exogenous or endogenous substance. For example, it is known that ratio of the amyloid non-producing pathway is increased by indirectly changing the metabolism of APP through the acceleration of phosphorylation of molecules other than APP caused by the activation of protein kinase C (PKC) (Racchi, M. (1999) *Trends Pharmacol. Sci.* 20, 418 - 423).

In recent years, it has been revealed that formation of Aβ and its accumulation in the brain are deeply concerned in the onset mechanism of AD. Its main research backgrounds are (1) Aβ induces nerve cell death in primary culture nerve cells, (2) it is considered that accumulation of senile plaque is an initial stage change of the pathological change in AD and (3) when mutation type APP gene or mutation type Presenilin 1 or 2 gene which can be found in familial AD is introduced into a host cell, acceleration of Aβ production or increase in the production ratio of Aβ₁₋₄₂ which has higher amyloid formation for Aβ₁₋₄₀ (*J. Biol*. *Chem.* (1996) 271, 18295 - 18298) are observed in comparison with the case of the introduction of wild type APP gene or wild type Presenilin 1 or 2 gene (*J. Biol. Chem.* (1996) 271, 18295 - 18298, *Science* (1997) 275, 630 - 631 and *Proc. Natl. Acad. Sci. USA* (1999) 96, 11049 - 11053).

Also, it is known that APP is phosphorylated in its intracellular domain (*Mol. Medicine* (1997) 3, 111 - 123). It has been shown that a species of Cdk, Cdc2 (Cdk1), is concerned in the phosphorylation of Thr668 of APP (the 668th position threonine residue of APP, the amino acid number is based on APP₆₉₅: APP (Thr668) hereinafter) in peripheral cells (non-nerve cells) (*EMBO J.* (1994) 13, 1114 - 1122). In recent years, it has been revealed that this residue is phosphorylated also in nerve cell or neuroblastoma such as PC12 cell and that the phosphorylation of this residue takes an important role in the function expression of APP in nerves (J. Neurosci. (1999) 19, 4421 - 4427).

The aforementioned Cdk has been discovered as serine/threonine kinase which controls cell cycle of eucaryote. Cdk does not show its activity by itself and requires binding with a control subunit called cyclin for its activation. In mammals, the presence of at least 8 species of Cdk have so far been known (*Protein, Nucleic Acid and Enzyme* (1997) 42, 1554 - 1561). Cdk5 has been discovered as a member of Cdk due to similarity of its primary structure (*EMBO J.* (1992) 11: 2909 - 2917), and it is known that it does not require cyclin for its activation but requires binding with subunits called p35 and p39 instead (Nature (1994) 371 (6496): 423 - 426, and *J. Biol. Chem.* (1995) 270: 26897 - 26903). These subunits are specifically expressed in nerve cells, and it is known that Cdk5 is concerned in functions of nerve cells, namely process elongation reaction in nerve cells and migration of nerve cells during the development process (*J. Neurosci.* (1999) 19, 6017 - 6026, *Genes & Dev.* (1996) 10, 816 - 825). That is, Cdk5 is not concerned in the control of cell cycle and its function is different from other Cdk though classified as Cdk. As the main substrates of Cdk5, APP, tau, neurofilament and the like are known (*J*. *Neurochem.* (2000) 75, 1085 - 1091, *Brain Res.* (1997) 765, 259 - 266, *J*. *Biol. Chem.* (1996) 271, 14245 - 14251, and *FEBS Lett.* (1993) 336, 417 - 424).

A large number of Cdk inhibiting substances are known as those which are concerned in the cell growth control, and there are reports stating that they are useful in neurodegenerative disease because of the action mechanism. In addition, these reports describe that said substances may be useful in AD by suppressing formation of a neurofibrillary change having phosphorylated tau as a composing element, based on an assumption that Cdk5 is concerned in the phosphorylation of tau (International Publication WO 99/07705, WO 99/02162, WO 99/65884, WO 99/30710, WO 99/62882 or WO 00/21550, and *J. Biochem.* (1995) 117, 741 - 749).

As described above, there are some reports on the relationship between Cdk, particularly Cdk5, and nerve cells and Aβ and AD and on the phosphorylation of APP by Cdk5, but there are no reports which suggest influence of Cdk on APP metabolism, particularly on the Aβ production. That is, there are no reports which suggest influence of a substance having Cdk inhibitory activity on the Aβ production.

### Disclosure of the Invention

As a result of intensive studies on the elucidation of the mechanism of dementia, particularly AD and the like, the present inventors have found that a substance having Cdk inhibitory activity can suppress Aβ production in nerve cells. Illustratively, as a result of comparative examination on the amount of Aβ production in the presence or absence of said Cdk inhibition substance in a system in which human APP gene is over-expressed in rat primary culture nerve cells, it was revealed that the substance having Cdk inhibitory activity reduces Aβ production. As a result of the achievement of Aβ production suppression, this invention is useful, for example, for the treatment of dementia, Alzheimer's disease and the like.

That is, the invention is a pharmaceutical composition for suppressing β-amyloid production which contains a substance having a cyclin-dependent kinase inhibitory activity as the active ingredient, preferably a pharmaceutical composition for suppressing β-amyloid production which contains a substance having a cyclin-dependent kinase 5 inhibitory activity as the active ingredient.

Also, the invention is a pharmaceutical composition for suppressing β-amyloid production, which is an anti-dementia drug or an anti-Alzheimer's disease drug.

Further, the invention is a pharmaceutical composition for suppressing β-amyloid production, which comprises a substance having an amyloid precursor protein threonine binding phosphorylation inhibitory activity as the active ingredient.

As another embodiment, the invention is a method for detecting β-amyloid production by allowing a substance having cyclin-dependent kinase inhibitory activity to contact with cells, preferably a method for detecting β-amyloid production wherein the cyclin-dependent kinase is cyclin-dependent kinase 5. Also, it is the above method for detecting β-amyloid production, which is a method for screening a β-amyloid production suppresser, and a kit for diagnosing dementia or Alzheimer's disease, which uses the method for detecting β-amyloid production. Also, the invention is a method for detecting β-amyloid production by allowing a substance having an amyloid precursor protein threonine binding phosphorylation inhibitory activity to contact with cells.

### Brief Description of the Drawings

Fig. 1 shows a result of analysis of total APP amount and degree of phosphorylation of APP (Thr668) by western blotting respectively using 22C11 antibody or phosphorylated APP (Thr668) antibody.
Fig. 2 shows calibration curves of Aβ₁₋₄₀ and Aβ₁₋₄₂ prepared using an Aβ solution having a concentration of from 0.2 to 10 ng/ml.
Fig. 3 shows degree of Aβ₁₋₄₀ production in the case of an Alsterpaullone treatment.
Fig. 4 shows degree of Aβ₁₋₄₂ production in the case of an Alsterpaullone treatment.
Fig. 5 shows degree of Aβ₁₋₄₀ production in the case of a Roscovitine treatment.

### Best Mode for Carrying Out the Invention

The following describes the invention in detail.

The "substance having cyclin-dependent kinase (Cdk) inhibitory activity" is a substance which keeps a serine/threonine kinase inhibition function that controls cell cycle of eucaryote or nerve cell functions such as process elongation reaction of nerves and migration of nerve cells during the development process, and which does not particularly require selectivity among Cdk subtypes but keeps inhibitory function at least against Cdk5 which is a Cdk subtype that functions in nerve cells. Examples of such compounds include those which are described in International Publication WO 97/0842, WO 97/16447, WO 98/33798, WO 98/50356, WO 99/07705, WO 99/02162, WO 99/09030, WO 99/15500, WO 99/030710, WO 99/34018, WO 99/62503, WO 99/65910, WO 00/01699, WO 00/12496, WO 00/21926, WO 00/18734, *Eur. J. Biochem.* (2000), 224, 771, *Eur. J. Biochem.* (2000), 267, 5983 and *Bioorganic* & *Med. Chem.* (1999), 7, 1281 and the like. Their typical examples include Roscovitine, Alsterpaullone or chemically modified products thereof (*J. Med. Chem.* (1999) 42, 2909 - 2919, and *Trends in Cell Biology* (1996) 6, 393 - 397). Also can be exemplified are commercial products or known compounds registered in Chemical File, compounds obtained by combinatorial chemistry techniques, culture supernatants of microorganisms, natural components derived from plants and marine organisms and animal tissue extracts, or antibodies and dominant negative mutant proteins. In addition, said substances whose substituents and the like are modified by chemical conversion which is a usual method for those skilled in the art can also be exemplified.

Illustratively, it is the aforementioned pharmaceutical composition for suppressing Aβ production which is a compound selected from a condensed heterocyclic ring derivative (I) represented by the following general formula or a pharmaceutically acceptable salt thereof, and Alsterpaullone (II) or a pharmaceutically acceptable salt thereof, 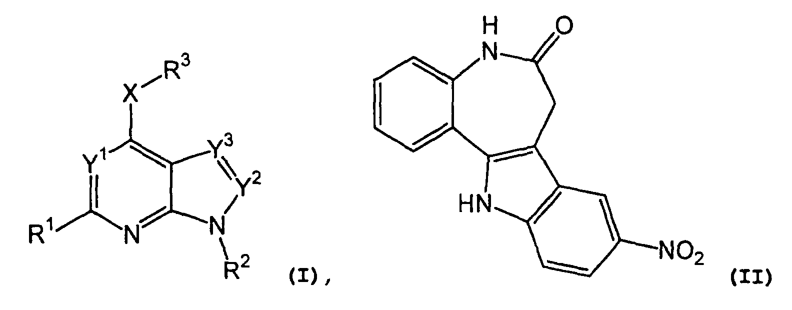 (R¹: H, halogen, OH, SH, hydrocarbon radical-S-, hydrocarbon radical-O-, NR⁴R⁵, hydrocarbon radical which may be substituted, heteroaryl which may be substituted or heterocyclic ring group which may be substituted
X: O, S, S(O)m, CH or NR⁶
m: 1 or 2
R², R³, R⁴, R⁵ and R⁶: H, OH, SH, hydrocarbon radical-S-, hydrocarbon radical-O-, hydrocarbon radical which may be substituted, hydrocarbon radical-O- which may be substituted, heteroaryl which may be substituted or heterocyclic ring group which may be substituted, which may be the same or different from one another
Y¹: N or CR⁷
Y²: N or CR⁸
Y³: N or CR⁹
R⁷, R⁸ and R⁹: H, OH, SH, heteroaryl which may be substituted, hydrocarbon radical which may be substituted, hydrocarbon radical-O- which may be substituted, hydrocarbon radical-S- which may be substituted, hydrocarbon radical-CO- which may be substituted, hydrocarbon radical-O-CO- which may be substituted or R¹⁰R¹¹NCO-, which may be the same or different from one another
R¹⁰ and R¹¹: H, hydrocarbon radical which may be substituted or hydrocarbon radical-O- which may be substituted, which may be the same or different from each other),
preferably the aforementioned pharmaceutical composition for suppressing Aβ production which is a purine derivative (III) represented by the following general formula or a salt thereof, 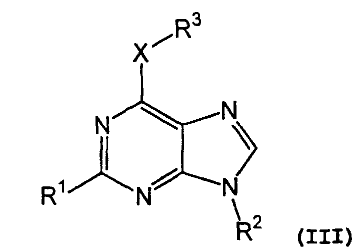 (R¹: H, OH, SH, hydrocarbon radical-O-, hydrocarbon radical-S- which may be substituted, NR⁴R⁵, hydrocarbon radical which may be substituted, heteroaryl which may be substituted or heterocyclic ring group which may be substituted
X: O, S, S(O)m, CH or NR⁶
m: 1 or 2
R², R³, R⁴, R⁵ and R⁶: H, OH, hydrocarbon radical which may be substituted, hydrocarbon radical-O- which may be substituted, heteroaryl which may be substituted or heterocyclic ring group which may be substituted, which may be the same or different from one another),
more preferably the aforementioned pharmaceutical composition for suppressing Aβ production which is a 1-aminopurine derivative represented by the following general formula or a salt thereof, 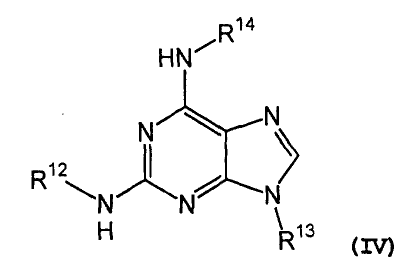 (R¹²: lower alkyl which may be substituted by OH or phenyl, or cycloalkyl or aryl which may be substituted by amino or OH
R¹³: lower alkyl or cycloalkyl
R¹⁴: lower alkyl, lower alkyl-O-lower alkyl or aryl or aralkyl which may be substituted by halogen, OH or lower alkyl-O-).
With the proviso that, even in the case of said substances, substances having non-selective influence on cells not originated from the Cdk inhibitory activity are excluded. Its example is a case in which said substances have an action to increase the Aβ amount for example by jointly having PKC inhibitory activity.

Symbols in the above general formulae are as follows.

As the "halogen", fluorine, chlorine, bromine, iodine or the like can be exemplified.

The "hydrocarbon radical" is a group of C₁₋₁₅, preferably C₁₋₁₀ straight or branched chain composed of carbon and hydrogen, and it illustratively means alkyl, alkenyl, alkynyl, cycloalkyl, cycloalkenyl, aryl, cycloalkyl-alkyl, cycloalkenyl-alkyl, aryl-alkyl, cycloalkyl-alkenyl, cycloalkenyl-alkenyl, aryl-alkenyl, cycloalkyl-alkynyl, cycloalkenyl-alkynyl or aryl-alkynyl.

The "alkyl" means a straight or branched saturated hydrocarbon radical, preferably a C₁₋₁₀ alkyl, and its illustrative examples include methyl, ethyl, isopropyl, hexyl, decyl and the like.

The "alkenyl" means a straight or branched hydrocarbon radical having at least one or more double bonds, preferably a C₂₋₁₀ alkenyl, and its illustrative examples include vinyl, propenyl, allyl, isopropenyl, hexenyl and the like.

The "alkynyl" means a straight or branched hydrocarbon radical having at least one or more triple bonds, preferably a C₂₋₁₀ alkynyl, and its illustrative examples include ethynyl, propynyl, butenyl and the like.

The "cycloalkyl" and "cycloalkenyl" mean monocyclic saturated and unsaturated hydrocarbon radicals, preferably a "C₃₋₈ cycloalkyl" and a "C₃₋₈ cycloalkenyl", and their illustrative examples include cyclopropyl, cyclopentyl, cyclohexyl, cyclopentenyl and the like.

The "aryl" means an aromatic hydrocarbon radical, preferably a C₆₋₁₄ aryl, and its illustrative examples include phenyl, tolyl, cumenyl, xylyl, naphthyl, biphenyl and the like.

The "cycloalkyl-alkyl", "cycloalkenyl-alkyl" and "aryl-alkyl" are groups in which hydrogen at an optional position of the aforementioned alkyl is substituted by the aforementioned cycloalkyl, cycloalkenyl and aryl, and their illustrative examples include cyclohexylmethyl, benzyl, phenethyl and the like.

The "heteroaryl" is a five- or six-membered monocyclic heteroaryl containing from 1 to 4 hetero atoms selected from N, S and O and a bicyclic heteroaryl condensed with benzene ring, which may be partially saturated. Furyl, thienyl, pyrrolyl, imidazolyl, pyrazolyl, thiazolyl, oxazolyl, pyridyl, pyrazinyl or pyrimidyl can be cited as the monocyclic heteroaryl, and benzofuranyl, benzothienyl, benzothiaziazolyl, benzothiazolyl, benzimidazolyl, indolyl, quinolyl or quinoxalinyl can be cited as the bicyclic heteroaryl. Preferred is a five- or six-membered monocyclic heteroaryl, particularly furyl, thienyl, imidazolyl, thiazolyl and pyridyl.

The "heterocyclic ring group" means a three- to seven-membered saturated or non-aromatic unsaturated ring group containing from 1 to 3 hetero atoms selected from nitrogen atom, oxygen atom or sulfur atom, and its examples include oxazolidinyl, tetrahydrofuranyl, 1,4-dioxanyl and tetrahydropyran.

As the "acyl", HCO-, C₁₋₂₅ hydrocarbon radical-CO-, C₁₋₂₅ hydrocarbon radical-CS-, heteroaryl-CO-, heteroaryl-alkyl-CO-, heteroaryl-alkenyl-CO-, heteroaryl-alkynyl-CO-, hetero ring-CO-, hetero ring-alkenyl-CO-, HCS-, heteroaryl-CS-, heteroaryl-alkyl-CS-, heteroaryl-alkenyl-CS-, heteroaryl-alkynyl-CS-, hetero ring-CS- or hetero ring-alkenyl-CS- can be cited. It is preferably a C₁₋₂₅ hydrocarbon radical-CO-, and its illustrative examples include formyl, acetyl, propionyl, 2-methylbut-2-enoyl, benzoyl and the like.

The "hydrocarbon radical-O-" means an alkoxy such as methoxy, ethoxy, phenoxy or the like.

The "esterificated carboxyl" means a "hydrocarbon radical-O-CO-" such as methoxycarbonyl, ethoxycarbonyl or the like. Groups substituted by the aforementioned acyl are also included in the esterificated carboxyl.

The substituent in the "hydrocarbon radical which may be substituted", "hydrocarbon radical-O- which may be substituted", "heteroaryl which may be substituted" or "heterocyclic group which may be substituted" is not particularly limited with the proviso that it is a group which can be substituted on these rings. These have 1 to 5 optional substituents which may be the same or different from one another.

Illustratively, it is a group selected from the following group A.

### Substituent A

Halogen; CN; NO₂; alkoxy; R^{a}R^{b}N- (R^{a} and R^{b}: the same or different from each other and each represents hydrogen atom, hydrocarbon radical, heteroaryl, heteroaryl-alkyl, hetero ring, hetero ring-alkyl or acyl (the same shall apply hereinafter)); hydrocarbon radical which may be substituted by esterificated carboxyl or R^{a}R^{b}N-; heteroaryl which may be substituted by hydrocarbon radical; hetero ring which may be substituted by hydrocarbon radical; acyl; D¹-G¹- (D¹: heteroaryl, hetero ring or R^{a}R^{b}N-, G¹: -O-, -S(O)ₙ-, -O-CO-, -CO-, -CS-, -O-CO-CO- or -CO-O-, n: 0, 1 or 2);
D²-G²- (D²: H, acyl-C₁₋₆ alkyl, heteroaryl-C₁₋₆ alkyl or hetero ring-C₁₋₆ alkyl, G²: -O-, -S(O)ₙ-, -O-CO-, -CO-, -CS-, -O-CO-CO- or -CO-O-);
D³-G³- (D³: hydrocarbon radical which may be substituted by alkoxy or esterificated carboxy, G³: -S(O)ₙ-, -O-CO-, -O-CO-CO- or -CO-O-).

The active ingredient of the invention has double bond so that it exists in geometrical isomer and tautomer forms. Isolated or mixed form these isomers are included in the invention.

Also, depending on the types of substituents, the compound of the invention may have asymmetric carbon atom so that isomers based on the asymmetric carbon can be present. Mixed or isolated forms of these optical isomers are included in the invention. In addition, compounds obtained by labeling the compound of the invention with radioactive isotopes are also included in the invention.

Further, the active ingredient of the invention may form acid addition salts or salts with bases depending on the type of substituents, and such salts are included in the invention so far as they are pharmaceutically acceptable salts. Their illustrative examples include acid addition salts with inorganic acids (e.g., hydrochloric acid, hydrobromic acid, hydriodic acid, sulfuric acid, nitric acid, phosphoric acid and the like) with organic acids (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid and the like), salts with inorganic bases (e.g., sodium, potassium, magnesium, calcium, aluminum and the like) and with organic bases (e.g., methylamine, ethylamine, ethanolamine, lysine, ornithine and the like), and ammonium salts and the like. Further, said substance may sometimes be isolated as hydrates, various types of solvates (e.g., ethanol solvate and the like) or as their polymorphic substances, and these various hydrates, solvates and polymorphic substances are also included in the invention.

Also, all of the compounds so called prodrugs which are metabolized and converted in the living body are included in the active ingredient of the invention. As the group which forms the prodrug of the invention, the groups described in *Prog. Med.,* 5, 2157 - 2161 (1985) and "Development of Medicaments" Vol. 7 (Hirokawa Shoten, 1990) Molecular Designing 163 - 198 and the like can be exemplified.

The "β-amyloid (Aβ)" is an amyloid substance which forms senile plaque. Said substance is also called β protein and formed by the degradation of APP by protease (processing) (Nature, (1987) 325, 733 - 736). Illustratively, Aβ means a fragment formed from APP during its intracellular secretion process after biosynthesis or a process in which it is incorporated from cell membrane and undergoes metabolism, by undergoing cutting by protease (processing), cutting by β-secretase Aβ domain conservatively and further undergoing cutting by γ-secretase (*Trends Pharmacol. Sci. 20,* 418 - 423, and *Proc. Natl. Acad. Sci.* USA (1999) 96, 11049 - 11053). This is mainly a protein called Aβ composed of 40 or 42 amino acid residues, namely Aβ₁₋₄₀ (SEQ ID NO;1) or Aβ₁₋₄₂ (SEQ ID NO;2), but other than this, variants thereof in which one or two or more amino acids are substituted, deleted or inserted can also be exemplified.

The "amyloid precursor protein: APP" means a precursor of the aforementioned Aβ and is a one transmembrane type membrane protein preferably having a short cytoplasmic domain composed of 47 amino acids or a domain in which one or two or more amino acids of said cytoplasmic domain may be substituted, deleted or inserted. It illustratively means each of three protein isoforms APP₆₉₅ (SEQ ID NO;3), APP₇₅₁ (SEQ ID NO;4) and APP₇₇₀ (SEQ ID NO;5), respectively composed of 695, 751 and 770 amino acids.

The phosphorylation of APP" means addition of phosphoric acid to APP, which occurs when APP undergoes metabolism in the living body for example during its intracellular secretion process, on the cell membrane or after its incorporation from the cell membrane. Though illustrative addition position of phosphoric acid to APP depends on the length of APP, in the case of APP₆₉₅, it means that the 668th threonine residue positioned in said APP intracellular domain (APP(Thr668)) (the amino acid number is based on APP₆₉₅) is phosphorylated (*Mol*. *Medicine* (1997) 3, 111 - 123).

The "substance having APP phosphorylation inhibitory activity" is a substance which inhibits phosphorylation of the 668th threonine residue positioned in the APP intracellular domain. Though it may be any substance which has said inhibitory activity, its illustrative examples include commercial products or known compounds registered in Chemical File, compounds obtained by combinatorial chemistry techniques, culture supernatants of microorganisms, natural components derived from plants and marine organisms and animal tissue extracts, or antibodies and dominant negative mutant proteins. In addition, said substances whose substituents and the like are modified by chemical conversion which is a usual method for those skilled in the art can also be exemplified.

The "dominant negative mutant" is a substance capable of specifically inhibiting activity of a protein by its competition with the binding of the protein with its substrate, a regulatory factor or the like, using a protein whose activity is deleted by introducing mutation into active site or activity regulating site of the protein, illustratively in the case of Cdk5, those in which the 144th asparagine residue as an important site for phosphoric acid transfer reaction was mutated into aspartic acid residue and the 33rd threonine residue as the ATP binding site was mutated into lysine residue are known as dominant negative mutant proteins (Genes & Development 10, 816 - 825 (1996)).

The "dementia" means a condition under which daily life and social life cannot be sufficiently made in various mental functions (e.g., memory, judgement, comprehension, language, capacity, cognition, feeling, volition, character and the like) due to cerebral organic disorders.

Though the "Alzheimer's disease (AD)" is sometimes strictly classified into AD which occurs at the middle-aged stage and senile dementia of type which occurs after 65 years or more of age, both cases are jointly called AD in this specification.

Accordingly, the "anti-dementia drug" or "anti-Alzheimer's disease drug" means a medicament for healing or improving the aforementioned diseases.

The "method for detecting β-amyloid production by allowing a substance having cyclin-dependent kinase (Cdk) inhibitory activity to contact with cells" or "method for detecting β-amyloid production by allowing a substance having an action to inhibit phosphorylation of threonine binding phosphoric acid of amyloid precursor protein to contact with cells" are described in detail in the following, and their examples include a kit for screening or diagnosing said substances and a test method for elucidating mechanism of AD. Preferred is a screening method of said substances.

The method of the invention for detecting Aβ production is described in detail.

### Preparation of vector DNA designed for expressing APP gene:

Structures of APP genes are preserved broadly from mammals to insects (*Proc. Natl. Acad. Sci. USA* (1992) 89, 10758 - 10762, *Proc. Natl. Acad. Sci. USA* (1989) 86, 2478 - 2482, *Biochem. J.* (1998) 330, 29 - 33, and *Proc. Natl. Acad. Sci. USA* (1993) 90, 12045 - 12049), and one or two or more APP genes of any APP species by which the invention can be carried out or mutants thereof in which amino acids are substituted, deleted or inserted are also included. Also included therein are mutation type APP found in familial AD (*Nature* (1991) 349, 704 - 706, *Science* (1991) 254, 97 - 99, *Nature* (1991) 353, 844 - 846, *Lancet* (1991) 337, 978 - 979, *Biochem. Biophys. Res. Comm.* (1991) 178, 1141 - 1146, *Lancet* (1991) 337, 1342 - 1343, and *Nature Genet.* (1992) 1, 343 - 347). Also, APP₆₉₅, APP₇₅₁ and APP₇₇₀ having different sequences due to difference in splicing are produced from the APP genes (*Biotechnology* (1989) 7, 147 - 163), and the APP as used herein includes all of these proteins produced from the APP genes.

As the vector DNA, any of known gene transfer vectors which do not exert influence for carrying out the invention can be used, including virus-derived vectors such as adenovirus vectors and the like (*Proc. Natl. Acad. Sci. USA* (1998) 95, 2509 - 2514) and expression vectors for mammal cells such as pEF-BOS (*Nucleic Acids Res.* (1990) 18, 5322), pSSR α (*Mol. Cell. Biol.* (1988) 8, 466 - 472) and the like. The promoter to be used in the expression of APP is not particularly limited, too.

### Cells and animals to be used in the test:

A nerve cell primary culture system can be used as the host cell. Preferably, the primary culture nerve cells are animal-derived cells, particularly mammal cells. For example, they are cells of a mammal generally used as an experimental animal, preferably rat or mouse, and preparation of the culture system is carried out in accordance with a known method (*Dev*. *Brain. Res.* (1986) 30, 47 - 56). It is desirable that the culturing period of the nerve cell primary culture system is the 7th day or more when the cells are sufficiently differentiated, preferably from the 7th to 10th day. A primary culture from an animal treated with gene manipulation is also included in said animal cells. For example, a cell strain established by separating from a temperature sensitive oncogene introduced transgenic mouse prepared by Taitoh et al. (a transgenic mouse expressing SV40 temperature sensitive T antigen by the SV40 self promoter (*Exp. Cell Res.* (1991) 197, 50 - 56) can also be exemplified as the host cell. Also can be exemplified are PC12 cell strain (J. Cell. Biol. (1978) 78, 747 - 755) and the like already established culture cell strains. The cells can be used are not particularly limited to nerve derived cells. In addition, cells of other than mammals can also be used, for example, *Drosophila* derived cells can be cited.

The APP gene can also be expressed transiently or stably in cells by using the calcium phosphate method (Virology (1973) 52, 456 - 467), Lipofectamine method (LIFE TECHNOLOGIES), FUGENE™ 6 method (BOEHRINGER MANNHEIM) and the like as the gene transfer method.

An expression vector designed such that expression of APP can be induced by a certain stimulation can also be expressed stably in cells. For example, in the case of the LacSwitch method (*Nucleic Acids Res.* (1991) 191, 4647 - 4653), expression of APP protein can be induced by adding IPTG (isopropyl-beta-D-thiogalactoside) to a medium. When expressed amount of endogenous APP is sufficient for measuring the amount of produced Aβ, it is possible to carry out the invention without particularly over-expressing exogenous APP.

In addition, an APP gene-introduced transgenic animal can also be used. Transgenic animals can be prepared by injecting an isolated gene into fertilized eggs and then transplanting the fertilized eggs into a false pregnancy animal to allow them to develop into individuals (*Science* (1981) 214, 1244 - 1246). In this case, Aβ production suppressing action of a compound to be tested can be observed at individual level. These transgenic animals can also be used for the detection of Aβ production suppressing action of a compound to be tested, by crossing them with a presenilin or Cdk5 over-expressing transgenic animal. In addition, the APP gene can also be introduced directly into an animal individual by a virus vector or the like. Those derived from viruses such as herpes simplex virus, adenovirus and the like can be used as the virus vector (*J. Neurosci.* (1996) 16, 486 - 496 and *J*. *Neurosci. Methods.* (1997) 71 77 - 84).

### Measurement of Aβ production:

It is known that Aβ is formed and released outside the cells even under normal conditions (*Nature* (1992) 359, 322 - 325, and *Science* (1992) 258, 126 - 129), and extracellular accumulation of Aβ is found also in the brain of AD. Accordingly, in the case of culture cells, it is desirable to carry out the Aβ production measuring method by measuring the amount of Aβ released in the culture medium to be used as the Aβ production. Though indirect, it is possible to measure the amount of Aβ which is present in the cells. In addition, in the case of a test using animal individuals including human, it is possible to measure the amount of Aβ in body fluids such as cerebrospinal fluid and the like and whole tissues. Accordingly, the detection method of the invention can be used in a diagnosing kit which measures the amount of Aβ by administering the pharmaceutical composition of the invention to patients.

Enzyme-linked immunosorbent assay (ELISA) and the like known methods are used for the measurement of the amount of Aβ. In the enzyme-linked immunosorbent assay, for example, an anti-Aβ antibody such as 6E10 (Wako) or the like is allowed to react with Aβ in a collected cell culture medium or Aβ₁₋₄₀ or Aβ₁₋₄₂ to be used as the standard, as the first step, this is allowed to react with an antibody such as R163, R165 (DR. PD Mehta of New York State Institute for Basic Research) or the like respectively capable of specifically binding to Aβ₁₋₄₀ or Aβ₁₋₄₂, as the second step, and then an appropriate anti-IgG antibody labeled with an enzyme (e.g., peroxidase or the like) is allowed to react therewith. The amount of Aβ in a culture medium is determined by measuring the activity of the enzyme (e.g., peroxidase or the like) (*Biochem. Biophys. Res*. *Commun.* (1972) 47, 846 - 851). Signal Select™ (BIOSOURCE), Human amyloid β (1-42) Measuring Kit (IBL) or the like commercially available kit can also be used in the ELISA method. In addition, determination methods by western blotting, dot blotting and the like can also be used (*Nature* (1970) 227, 680 - 685, and *Proc. Natl. Acad. Sci. USA* (1979) 76, 4350 - 4354).

### Evaluation of compounds to be tested:

By the above methods, a method for the detection of Aβ production suppressing action based on Cdk, preferably Cdk5, inhibitory activity using a large number of compounds to be tested, or a method for the detection of Aβ production suppressing action based on the inhibition of APP threonine phosphate phosphorylation, can be cited. For example, a compound to be tested is added to a cell culture medium to a final concentration of 1 nM to 200 µM, and after a predetermined period of time, the culture medium is collected to determine the produced amount of Aβ by the aforementioned method. The Cdk5 inhibitory activity can be determined by allowing Cdk5 purified from an animal cell or animal tissue by immunoprecipitation or using an appropriate column or recombinant Cdk5 or p25 expressed in and purified from Sf9 cell or the like insect cell (p35 or p39 or respective active site fragment thereof, p21, N145, p30 or the like can be used (*Nature* (1994) 371 (6496): 423 - 426, *J. Biol. Chem.* (1997) 272: 12318 - 12327, and *J. Biol. Chem.* (1995) 270: 26897 - 26903) to react with isotope-labeled ATP and an appropriate protein or peptide to be used as the substrate in an appropriate buffer, and then measuring amount of the isotope incorporated into the substrate by so-called scintillation proximity kinase assay (SPA method) or autoradiography (*Eur. J. Biochem.* (1997) 243, 527 - 536, and Biochem. (1999) 268, 318 - 329). The degree of APP phosphorylation can be determined after solubilization of cells using a solubilizing agent, for example by western blotting method using an antibody which specifically react with phosphorylated state of APP (*J. Neurosci.* (1999) 19, 4421 - 4427).

As the compounds to be tested which can be evaluated by the method of the invention, commercial products or known compounds registered in Chemical File and compounds obtained by combinatorial chemistry techniques can be used. In addition, culture supernatants of microorganisms, natural components derived from plants and marine organisms, animal tissue extracts and the like can also be used. Also, antibodies, dominant negative mutant proteins and the like can be used, too. Also useful are chemically modified products of substances found by the method of the invention.

The following describes the pharmaceutical composition of the invention for suppressing Aβ production in detail.

The pharmaceutical composition of the invention for suppressing Aβ production is prepared into tablets, powders, fine subtilaes, granules, capsules, pills, solutions, injections, suppositories, ointments, adhesive preparations and the like using generally used pharmaceutical carriers, fillers and other additives and administered orally (including sublingual administration) or parenterally.

Clinical dose of the pharmaceutical composition of the invention for suppressing Aβ production in human is optionally decided by taking into consideration symptoms, weight, age, sex, route of administration and the like of each patient to be treated, but is usually within the range of from 0.1 mg to 5,000 mg, preferably from 1 mg to 500 mg, per day per adult by oral administration, once a day or dividing the daily dose into several doses, or within the range of from 0.1 mg to 5,000 mg, preferably from 1 mg to 500 mg, per day per adult by parenteral administration, once a day or dividing the daily dose into several doses, or by intravenous continuous administration within the range of from 1 hour to 24 hours per day. As a matter of course, since the dose varies under various conditions as described in the foregoing, a smaller dose than the above range may be sufficient enough in some cases.

As the dosage form of the pharmaceutical composition of the invention for suppressing Aβ production, tablets, powders, granules and the like are used. In such dosage forms, one or more active substances are mixed with at least one inert diluent such as lactose, mannitol, glucose, hydroxypropylcellulose, microcrystalline cellulose, starch, polyvinyl pyrrolidone or aluminum magnesium silicate. In the usual way, the composition may contain other additives than the inert diluent, such as a lubricant (e.g., magnesium stearate or the like), a disintegrating agent (e.g., calcium cellulose glycolate or the like), a stabilizing agent (e.g., lactose or the like) and a solubilization assisting agent (e.g., glutamic acid, aspartic acid or the like). If necessary, tablets or pills may be coated with a film of a gastric or enteric substance such as sucrose, gelatin, hydroxypropylcellulose, hydroxypropylmethylcellulose phthalate or the like. Said dosage forms further include pharmaceutically acceptable emulsions, solutions, suspensions, syrups, elixirs and the like, and contain a generally used inert diluent such as purified water or ethanol. In addition to the inert diluent, this composition may also contain auxiliary agents such as a solubilizing or solubilization assisting agent, a moistening agent, a suspending agent and the like, as well as sweeteners, flavors, aromatics and antiseptics.

The injections for parenteral administration includes aseptic aqueous or non-aqueous solutions, suspensions and emulsions. Examples of the diluent for use in the aqueous solutions and suspensions include distilled water for injection and physiological saline. Examples of the diluent for use in the non-aqueous solutions and suspensions include propylene glycol, polyethylene glycol, plant oils (e.g., olive oil or the like), alcohols (e.g., ethanol or the like), polysorbate 80 (trade name) and the like. Such a composition may further contain additive agents such as a tonicity agent, an antiseptic, a moistening agent, an emulsifying agent, a dispersing agent, a stabilizing agent (e.g., lactose) and a solubilizing or solubilization assisting agent. These compositions are sterilized by filtration through a bacteria retaining filter, blending of a germicide or irradiation. Alternatively, they may be used by firstly making into sterile solid compositions and dissolving them in sterile water or a sterile solvent for injection prior to their use.

In order to disclose the invention further illustratively, its examples are described in the following, though the invention is not limited the examples.

### (Example 1)

### Quantity of APP phosphorylation in rat hippocampus primary culture nerve cells and construction of Aβ production measuring system:

### (1) Preparation of APP adenovirus vector:

A human APP₆₉₅ gene encoding 695 amino acids (SEQ ID NO;6) was used as the APP gene. An adenovirus vector was prepared in accordance with the ADEasy system (*Proc. Natl. acad. Sci.* USA (1998) 95, 2509). In order to add CAG promoter, after blunt-end treatment of termini, the APP₆₉₅ gene was once introduced into *Hinc*II site of pCAG-pA (*Gene* (1991) 108, 193 - 200), and then the CAG promoter and its downstream APP gene were cut out using *Pst*I and *Xho*I sites, blunt-ended and then introduced into the *Eco*RV site of a shuttle vector, pADTrack, of the ADEasy system. The APP gene-introduced shuttle vector was converted into linear strand using PmeI and introduced into an Escherichia coli strain BJ5183 together with pADEasy-2 vector to prepare a viral genome recombinant plasmid.

### (2) Preparation of adenovirus:

The APP gene-containing recombinant adenovirus vector was converted into linear strand by *Pac*I treatment and introduced into HEK293 cells by the Lipofectamine method (LIFE TECHNOLOGIES), and 3 to 5 days thereafter when cells were pealed off from the plate, all of the cells were recovered. The recombinant adenovirus was obtained from the supernatant by repeating freezing-thawing step of the cells. The thus obtained recombinant adenovirus was prepared in a large amount by repeating infection of HEK293 cells and then concentrated and purified by the CsCl method and used in the test.

### (3) Preparation of rat primary culture nerve cells:

A pregnant rat was anesthetized with diethyl ether and allowed to cause death due to hemorrhage by thoracic (heart) incision, and then the womb was extracted by incising the abdominal side. The fetus was sterilized with ethanol for disinfection in a clean bench, the total brain was extracted and recovered in a dish containing a serum-free medium (SUMILON, Sumitomo Bakelite), and the hippocampus was extracted using a precision *pincette* under a stereoscopic microscope. The hippocampus was transferred into a 50 ml tube, tissues were precipitated by standing to discard the supernatant by aspirating and a cell dispersing solution (PBS containing 1% papain, 150 U/ml DNase I, 0.02% L-cysteine, 0.02% BSA and 0.5% glucose) was added thereto, and the mixture was incubated at 37°C for 15 minutes and then centrifuged (1,000 rpm, 4°C, 5 min). The supernatant was removed by aspiration, 10 ml of the serum-free medium was added to the sediment, pipette treatment was carried out several times, and then cell masses were removed using a filter to obtain a cell suspension. The number of cells was counted using trypan blue, and 1.5 x 10⁵ of the cells were inoculated into a poly-L-lysine-coated 48 well culture plate. In order to effect sufficient differentiation, they were cultured at 37°C for 10 days in a CO₂ incubator and then used in the test.

### (4) Infection of primary culture nerve cells with adenovirus:

Infection with adenovirus was carried out on the 10th day of the culturing. A virus suspension of 1.5 x 10⁸ particles was suitably diluted and added to a medium to an MOI (multiplicity of infection) value of 1,000 based on 1.5 x 10⁵ cells. A sample was recovered on the 4th day of the infection.

### (5) Measurement of APP phosphorylation:

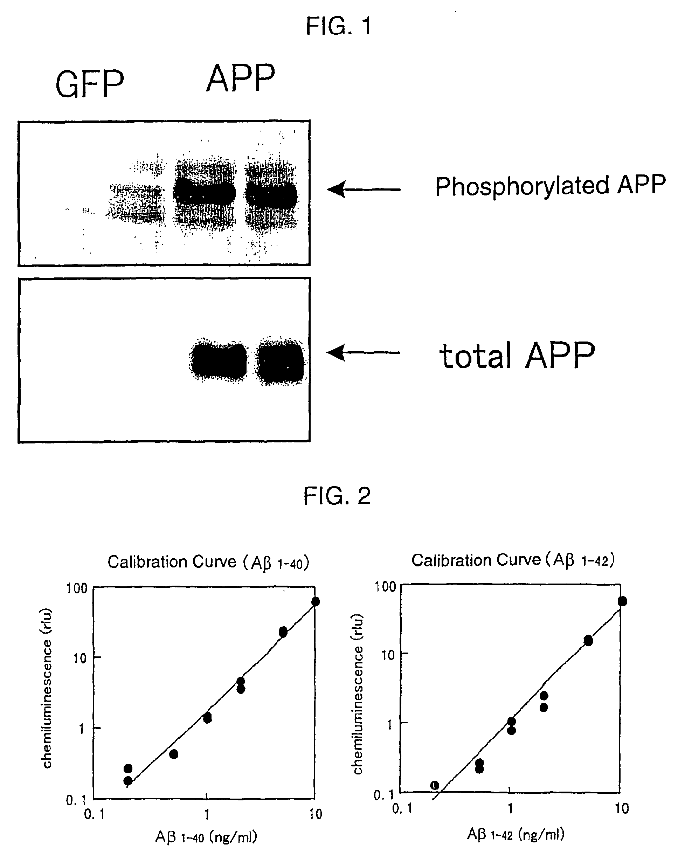

A 50 µl portion of cell lysis solution (50 mM Tris-HCl, 1% SDS, 2.7 M urea, 1 mM Na₃VO₄, 1 mM NaF) was added to 1.5 x 10⁵ cells, the mixture was recovered in a tube, subjected to about 5 seconds of ultrasonic disintegration and then centrifuged (15,000 rpm, 5 min), and the thus obtained supernatant was used as a sample. A portion of the sample was separated by SDS-PAGE (7.5% polyacrylamide gel) and then transferred onto a PVDF (polyvinylidene difluoride) membrane (Daiichi Pure Chemicals) using a semi-dry blotting apparatus (BIO-RAD). The thus obtained PVDF membrane was soaked in Block Ace (Daiichi Pure Chemicals) at 4°C overnight to carry out blocking. A polyclonal antibody (anti-phosphorylation APP(Thr668) antibody) for use in the detection of the phosphorylation of APP(Thr668) was prepared by preparing a peptide in which phosphate group was introduced into the T residue of an amino acid sequence NH₂-AAVTPEERHC (SEQ ID NO;7) corresponding to the intracellular region of APP, binding it to KLH, and then immunizing a rabbit therewith (*J. Neurosci.,* 19, 4421 - 4427 (1999)). The anti-serum obtained by the immunization was used by subjecting to adsorption treatment using an immunogen peptide before introduction of phosphate group and then purifying by affinity chromatography using the phosphate group-introduced antigen peptide. Also, an anti-APP monoclonal antibody 22C11 (Roche Molecular Biochemicals) was used in the determination of total APP amount (combined APP amount of phosphorylated state of APP and non-phosphorylated state of APP). The PVDF membrane after blocking was lightly washed with PBS-T (PBS (140 mM NaCl, 10 mM phosphate buffer, pH 7.4) containing 0.05% Tween 20), soaked in a 1,000 times diluted primary antibody (anti-phosphorylation APP(Thr668) antibody or 22C11) solution and allowed to undergo the reaction at room temperature for 1 hour. After completion of the reaction, washing with PBS-T at room temperature for 10 minutes was repeated four times and then this was subjected to the reaction with a secondary antibody. As the secondary antibody, 20,000 times diluted HRP-labeled anti-rabbit and anti-mouse IgG antibodies (Amersham Pharmacia Biotech) were used. In the same manner as the case of the primary antibody reaction, they were allowed to undergo the reaction at room temperature for 1 hour and then subjected to washing with PBS-T in the same manner. ECL-plus detection kit (Amersham Pharmacia Biotech) was used in the detection of the reaction, and analysis of chemiluminescence was carried out by Storm860 Imageanalyzer (Amersham Pharmacia Biotech) and autoradiography. Fig. 1 shows a result of analysis of total APP amount and degree of phosphorylation of APP (Thr668) by western blotting respectively using 22C11 antibody or phosphorylated APP (Thr668) antibody, after infection of primary culture nerve cells with wild type APP₆₉₅ adenovirus. As a control, the cells were infected with adenovirus which had been integrated with GFP (jellyfish green fluorescent protein, *Neuron* (1996) 16, 255 - 260) (SEQ ID NO;8). Significant increase in the total APP and phosphorylation of APP (Thr668) was observed by the infection with wild type APP₆₉₅ adenovirus.

### (6) Measurement of Aβ production:

Culture supernatants recovered 24 hours after the addition of compounds to be tested were used as the samples for Aβ production measurement. Amount of Aβ in each supernatant was determined by sandwich ELISA. Firstly, an anti-Aβ monoclonal antibody 6E10 (Wako) diluted to 10 µg/ml with 100 mM phosphate buffer was dispensed in 50 µl/well portions into a plate for ELISA (Maxisorp, Nunc) and allowed to stand overnight at 4°C to effect adhesion of the 6E10 antibody to the plate. The antibody was recovered on the next day, and 200 µl of Block Ace was added to each well to carry out blocking at 4°C overnight or more. The plate was washed 4 times with TBS-T (TBS (20 mM Tris pH 7.5, 150 mM NaCl) containing 0.05% Tween 20) using a plate washer (Bio-Rad, Model 1250 Immuno Wash), and then a sample diluted 2 times with Block Ace or Aβ for calibration curve preparation was added and allowed to undergo the reaction at 4°C overnight. The Aβ for calibration curve preparation was prepared in the following manner. Each of Aβ₁₋₄₀ (SEQ ID NO;1) and Aβ₁₋₄₂ (SEQ ID NO;2) was purchased from California Peptide and dissolved in hexafluoro-2-propanol (HFIP, Kanto Kagaku) to a concentration of 1 mg/ml to be used as a stock solution. A 10 µl portion of the stock solution was transferred into a tube, HFIP was evaporated using a vacuum concentrator and then the residue was again dissolved in 10 µl of DMSO to an Aβ concentration of 1 mg/ml. Serial dilutions of the solution for Aβ calibration curve measurement were prepared within the range of from 0.2 to 10 ng/ml. The plate after the reaction with a sample or Aβ for calibration curve preparation was washed 4 times with the plate washer and then allowed to react with primary antibodies. Polyclonal antibodies R163 (Aβ₁₋₄₀ specific antibody) and R165 Aβ₁₋₄₂ specific antibody), which specifically recognize C-terminus of Aβ, were used as the primary antibodies. R163 and R165 were purchased from DR. PD Mehta (New York State Institute for Basic Research) and purified as IgG fraction. Each antibody was diluted 1,000 times with TBS-T containing 25% Block Ace, dispensed in 60 µl/well portions into the plate and then allowed to undergo the reaction at room temperature for 2 hours. After the reaction, the plate was washed 4 times using the plate washer and then the reaction with a secondary antibody was carried out. An HRP-labeled anti-rabbit IgG antibody was used as the secondary antibody, diluted 8,000 times with TBS-T containing 25% Block Ace and dispensed in 60 µl/well portions, and then the reaction was carried out at room temperature for 2 hours. After completion of the reaction, the plate was washed 4 times using the plate washer and then chemiluminescence by a POD chemiluminescence reagent (Boehringer Mannheim) was measured by ML3000 Plate Reader (Dynatech Laboratories). Fig. 2 shows calibration curves of Aβ₁₋₄₀ and Aβ₁₋₄₂ prepared using an Aβ solution adjusted to a concentration of from 0.2 to 10 ng/ml.

### (Example 2)

### Measuring method of Cdk5 activity and evaluation of Cdk5 inhibitory activity of Alsterpaullone and Roscovitine

### (1) Preparation of Cdk5 and p25:

Regarding the active site of Cdk5 gene (*EMBO J.*, 11(8), 2909 - 2917 (1992)) and p35 gene (*Nature,* 371(6496), 419 - 423 (1994)), namely a region corresponding to p25, its human type was obtained by RT-PCR. Complete length of the human Cdk5 gene was amplified from total RNA prepared from a human neuroblastoma SH-SY5Y using a primer set of 5'-TACGGATCCGCAGAAATACGAGAAACTGG-3' (SEQ ID NO;9) and 5'-CTGAAGGTTTAGGGCGGACAGAAGTCGGAG-3' (SEQ ID NO; 10). The total RNA was obtained by adding 1 ml of ISOGEN reagent (Nippon Gene) to 1 x 10⁶ cells, mixing the resulting lysate with 1/5 volume of chloroform, centrifuging the mixture at 15,000 rpm for 10 minutes, mixing the resulting supernatant with 1/2 volume of 2-propanol and further centrifuging the mixture at 15,000 rpm for 20 minutes. The thus obtained RNA was dissolved in water and, after measurement of its concentration, subjected to PCR reaction (Titan™ one tube RT-PCR kit (Boehringer Mannheim)). Also, the active site of human p35 gene, namely a region corresponding to p25, was amplified in the same manner using a primer set of 5'-TACGGATCCCCAGGCGTCCACCAGTGAG-3' (SEQ ID NO;11) and 5'-TACAAGCTTCATGACGCAGGCTACAGTGC-3' (SEQ ID NO;12). Each of the genes was introduced into a Bacmid preparation vector pFASTBac-Hta (Gibco BRL) which had been designed such that His-tag is added to N-terminus, making use of the *Hind*III and *Bam*HI sites designed in the primers. Each of the genes introduced into pFASTBac was introduced into DH10Bac to carry out recombination in *E. coli,* thereby obtaining respective recombinant Bacmids. The thus obtained Bacmid was introduced into Sf9 cells using cellfectin reagent (Gibco BRL) to obtain baculovirus. By infecting Sf9 cells with the thus obtained baculovirus, recombinant Cdk5 protein (SEQ ID NO;13: the Cdk5-originated sequence is in and after the 28th position amino acid) and p25 protein (SEQ ID NO;14: the p25-originated sequence is in and after the 28th position amino acid) produced by the baculovirus were obtained. Purification of the recombinant proteins was carried out making use of the 6X His tag added to the N-terminus.

That is, the Sf9 cells infected with baculovirus were lysed using a cell lysis solution (50 mM Tris-HCl, 10 mM 2-ME, 1mM PMSF, 1% NP-40), subjected to ultrasonic disintegration and then centrifuged (10,000 x g, 30 min), and the thus obtained supernatant was applied to Ni-NTA column (QIAGEN) which had been equilibrated with a column buffer solution A (20 mM Tris-HCl, 500 mM KCl, 20 mM imidazole, 10 mM 2-ME, 10% glycerol). After though washing with the buffer A, this was washed with a buffer B (20 mM Tris-HCl, 1 M KCl, 10 mM 2-ME, 10% glycerol). Finally, fractions eluted with a buffer C (20 mM Tris-HCl, 100 mM KCl, 100 mM imidazole, 10 mM 2-ME, 10% glycerol) were recovered, dialyzed against a sample solution (50 mM HEPES-KOH, 10 mM MgCl₂, 2.5 mM EGTA, 0.1 mM PMSF) and used as a purified preparation.

### (2) Measurement of Cdk5 activity:

A 1:1 mixture of the purified Cdk5 protein and purified p25 obtained above was used as the enzyme source at the time of kinase activity measurement. Histone H1 (Sigma) was biotinylated and used as the substrate. Biotinylated histone was prepared by allowing histone to react with 10 equivalents of biotin (Ez-link Sulfo-NHS-LC-Biotin, Pierce) as molar ratio in the presence of 50 mM sodium bicarbonate at room temperature for 6 hours. Free biotin was removed by carrying out dialysis using TBS, and the resulting solution was used as the stock solution. Measurement of kinase activity was carried out in accordance with the SPA method (Amersham). The substrate, enzyme and γ ³³P-ATP were allowed to undergo the reaction at room temperature for 1 hour in a kinase reaction solution (50 mM HEPES-KOH, 10 mM MgCl₂, 2.5 mM EGTA, 0.1 mM PMSF, 1 mM Na₃VO₄, 1 mM NaF, 5 µg/ml aprotinin), and then the reaction was terminated by adding 3 volumes of a reaction termination solution (PBS containing 50 mM ATP, 5 mM EDTA, 1% Triton X-100 and 15 mg/ml streptavidin SPA beads) and stirring the mixture. This was allowed to stand at room temperature for 15 minutes and centrifuged (1,700 rpm, 2 min), and then amount of the isotope incorporated in the substrate was measured by Topcount (Beckman).

### (3) Measurement of kinase inhibitory activity of Alsterpaullone, Roscovitine and compounds A, B, C and D:

Though chemical structures of Alsterpaullone and Roscovitine are completely different from each other, it is known that both are inhibitors selective for Cdk (Cancer Res. (1999) 59, 2566 - 2569, *J. Med. Chem.* (1999) 42, 2909 - 2919, *Eur. J. Biochem.* (1997) 243, 518 - 526, *Eur. J. Biochem.* (1997) 243, 527 - 536, and WO 99/65910). It is known that the compounds A, B, C and D are Cdk inhibitors (*Bioorganic* & *Medicinal Chemistry Letters* (1999) 9, 91 - 96, WO 97/16452, WO 99/43675). The inventors have verified the Cdk5 inhibitory activity of said compounds by the aforementioned method.

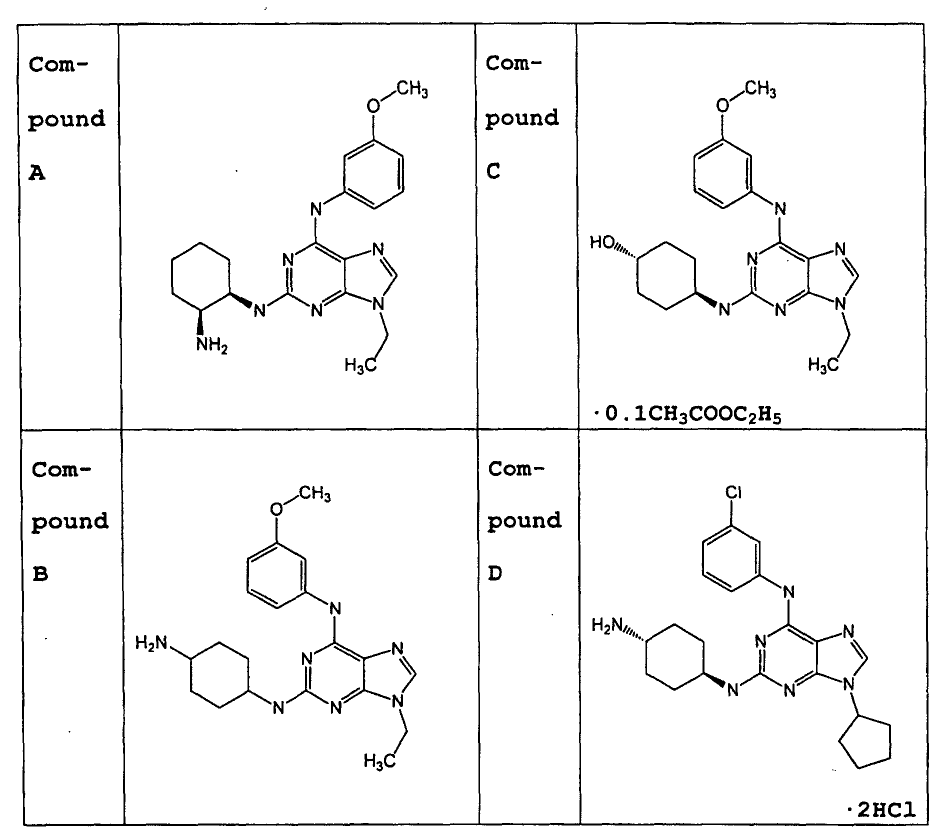

### (Example 3)

### Influence of Alsterpaullone, Roscovitine and compounds A, B, C and D on the amount of APP phosphorylation:

Hippocampus primary culture nerve cells on the 10th day of culturing were infected with APP₆₉₅ adenovirus, and 3 days thereafter, the medium was exchanged with a medium to which an appropriate concentration of Alsterpaullone (DMSO solution) or Roscovitine (DMSO solution) had been added. After additional 1 day of culturing, the cells were recovered using a cell lysis solution, and the degree of APP(Thr668) phosphorylation was determined by the method shown in Example 1. For example, the APP phosphorylation suppressing action of Alsterpaullone and Roscovitine was found at 10 µM and 50 µM, respectively. In this case, no changes were found in the expressed amount of total APP when detected with the anti-APP antibody 22C11. The APP phosphorylation suppressing action was also found in the compounds A, B, C and D in the same manner. In addition, said action was found also in a compound E or F having completely different structure from those of the aforementioned compounds.

### (Example 4)

### Influence of Alsterpaullone on the Aβ production:

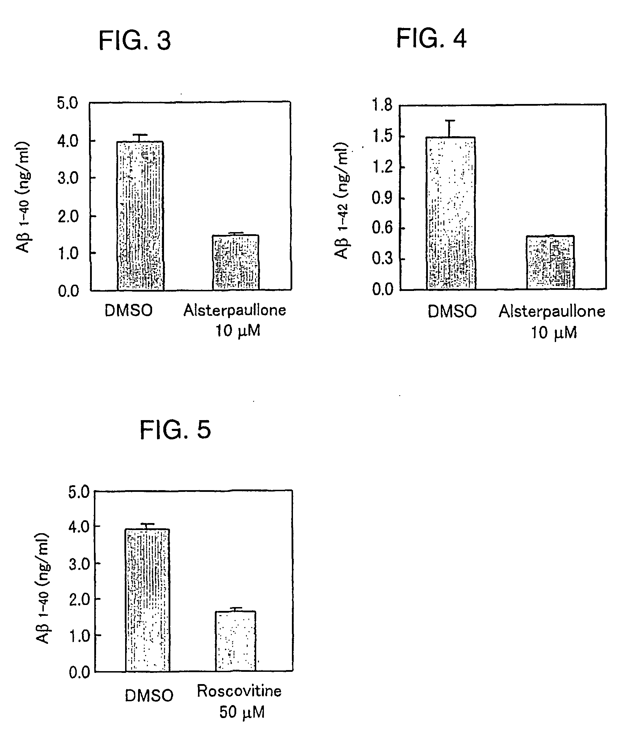

Hippocampus primary culture nerve cells on the 10th day of culturing were infected with APP₆₉₅ adenovirus, and 3 days thereafter, the medium was exchanged with a medium to which an appropriate concentration of Alsterpaullone (DMSO solution) had been added. After additional 1 day of culturing, the culture supernatant was recovered, and the amount of Aβ in the supernatant was determined by the method shown in Example 1. Alsterpaullone suppressed Aβ₁₋₄₀ production (Fig. 3) and Aβ₁₋₄₂ production (Fig. 4). As a control, DMSO was used instead of Alsterpaullone.

### (Example 5)

### Influence of Roscovitine and compounds A, B, C and D on Aβ₁₋₄₀ production:

In the same manner as in Example 4, amount of Aβ1-40 in the supernatant was determined. The Aβ production suppressing effect of Roscovitine was confirmed (Fig. 5). The Aβ production suppressing effect of the compounds A, B, C and D was also confirmed. In addition, said effect was also found in the aforementioned compounds E and F.

Thus, from the tests using substances having the Cdk inhibitory action but completely different chemical structures, it was found that said substances can reduce the Aβ production. In addition, based on these test results and the fact that the Cdk isozyme which is known to have a physiological function in nerve cells is Cdk5 (*J*. *Neurosci.* (1999) 19, 6017 - 6026, and *Genes & Dev*. (1996) 10, 816 - 825), it was confirmed that said compounds exerted the Aβ production suppressing action by inhibiting the activity of Cdk5.

### (Example 6)

### Influence of Cdk5 dominant negative mutant adenovirus on Aβ production

### (1) Preparation of Cdk5 dominant negative mutant adenovirus vector

A dominant negative mutant of Cdk5 (prepared by converting the 144th position aspartic acid residue into asparagine residue: Cdk5 D144N hereinafter (SEQ ID NO;15)) was prepared based on the description of Nikolic *et al.* (*Genes* & *Development,* 10, 816 - 825 (1996)). Quick Change Kit (Stratagene) was used in the introduction of mutation, and the actual operation was carried out in accordance with the instructions. As preparation for introducing a mutation, the Cdk5 gene described in Example 2 was used as the template, again amplified by PCR using a primer set of 5'-CTGAAGCTTCGCAGAAATACGAGAAACTGG-3' (SEQ ID NO;16) and 5'-GATCTCGAGTAGGGCGGACAGAAGTCGGAG-3' (SEQ ID NO;17) and introduced into pGEM-T Easy vector (Promega). The Cdk5 gene introduced into pGEM-T Easy vector was used as the template and amplified by PCR using a primer set of 5'-GGAGCTGAAATTGGCTAATTTTGGCCTGGCTCG-3' (SEQ ID NO;18) and 5'-CGAGCCAGGCCAAAATTAGCCAATTTCAGCTCC-3' (SEQ ID NO;19), and the product was treated with *Dpn*I at 37°C for 3 hours and then introduced into an *E. coli* strain JM-109 to obtain a mutation-introduced gene. After confirmation of its nucleotide sequence, in order to re-add the initiation methionine, the mutation gene was used as the template and amplified by PCR using a primer set of 5'-CTGAAGCTTATGCAGAAATACGAGAAACTGG-3' (SEQ ID NO;20) and 5'-GATGTCGACTAGGGCGGACAGAAGTCGGAG-3' (SEQ ID NO;21), and then cut out using the *Hin*dIII and *Sal*I sites designed in the primers and introduced into a shuttle vector of ADEasy system, pShuttle. The pShuttle was used by introducing in advance a region of from CAG promoter to poly(A) addition signal (from *PstI* site to *Xho*I site) of the pCAG-pA vector described in Example 1. In order to facilitate insertion of the gene in carrying out the introduction, a multi-cloning site (a region which is cut out with *Bss*HII) derived from pBluescript II-KS (Stratagene) was introduced into the *Hinc*II site existing between the CAG promoter and poly(A) addition signal. The shuttle vector introduced with the dominant negative mutant Cdk5 was made into linear strand using *Pme*I and introduced into an *E. coli* strain BJ5183 together with pADEasy-2 vector to prepare a virus genome recombinant plasmid. Preparation of adenovirus was carried out in the same manner as the case of APP adenovirus shown in Example 1.

### (2) Influence of Cdk5 D144N adenovirus on Aβ production

Hippocampus primary culture nerve cells on the 7th day of culturing were infected with wild type APP₆₉₅ adenovirus together with the Cdk5 D144N adenovirus, the culture supernatant 2 days thereafter was recovered, and the amount of Aβ in the supernatant was determined. In comparison with a control, Cdk5 D144N suppressed the Aβ₁₋₄₀ production. As the control, cells infected with GFP-integrated adenovirus were used and compared in the same manner as in Example 1. Thus since similar Aβ production inhibitory activity found in the Cdk inhibitors was observed in the dominant negative mutant of Cdk5 capable of specifically suppressing the function of Cdk5, it was confirmed that the Aβ production can be suppressed by inhibiting the activity of Cdk5.

### Industrial Applicability

Suppression of Aβ production can be achieved by the pharmaceutical composition of the invention. A disease based on said production suppression (e.g., AD) is characterized by pathological changes such as falling off of nerve cells, reduction of synapse numbers, senile plaque in the brain, accumulation of neurofibrillary change and the like, and senile plaque in the AD brain among them, namely accumulation of Aβ as the main composing molecule of the amyloid protein of senile plaque, is deeply concerned in the onset mechanism of AD, so that the invention is useful for anti-dementia drugs, anti-Alzheimer's disease drugs and the like.

Also, by detecting Aβ production by allowing a substance having Cdk, preferably Cdk5, inhibitory activity or a substance having APP threonine binding phosphoric acid phosphorylation inhibitory activity to contact with cells making use of the invention, for example, a substance having Aβ production suppressing action can be screened, and it can be used in diagnosis kits for dementia, Alzheimer's disease and the like.

## Claims

1. A pharmaceutical composition for suppressing β-amyloid production, which comprises a substance having cyclin-dependent kinase inhibitory activity as the active ingredient.

2. A pharmaceutical composition for suppressing β-amyloid production, which comprises a substance having cyclin-dependent kinase 5 inhibitory activity as the active ingredient.

3. The pharmaceutical composition for suppressing β-amyloid production according to claim 1 or 2, wherein it is an anti-dementia agent or anti-Alzheimer's disease drug.

4. A pharmaceutical composition for suppressing β-amyloid production, which comprises a substance having amyloid precursor protein threonine binding phosphorylation inhibitory activity as the active ingredient.

5. A method for detecting β-amyloid production by allowing a substance having cyclin-dependent kinase (Cdk) inhibitory activity to contact with cells.

6. The method for detecting β-amyloid production according to claim 5, wherein the cyclin-dependent kinase (Cdk) is cyclin-dependent kinase 5 (Cdk5).

7. A method for detecting β-amyloid production by allowing a substance having amyloid precursor protein threonine binding phosphorylation inhibitory activity to contact with cells.

8. A method for screening a substance having β-amyloid production suppressing action using the method for detecting β-amyloid production described in claims 5 to 7.

9. A diagnosing kit for dementia or Alzheimer's disease, which uses the method for detecting β-amyloid production described in claims 5 to 7.

10. A method for treating dementia or Alzheimer's disease, which comprises administering the pharmaceutical composition for suppressing β-amyloid production described in claims 1 to 4.


SEQUENCE LISTING

<110> YAMANOUCHI PHARMACEUTICAL CO., LTD.
      TOSHIHARU, SUZUKI

<120> COMPOSITION FOR SUPPRESING A PRODUCT OF AMYLOID BETA

<130> Y0111-PCT

<140>
<141>

<150> JP2000131037
<151> 2000-04-28

<160> 24

<170> PatentIn Ver. 2.1

<210> 1
<211> 40
<212> PRT
<213> Homo sapiens

<400> 1
Asp Ala Glu Phe Arg His Asp Ser Gly Tyr Glu Val His His Gln Lys
  1               5                  10                  15

Leu Val Phe Phe Ala Glu Asp Val Gly Ser Asn Lys Gly Ala Ile Ile
             20                  25                  30

Gly Leu Met Val Gly Gly Val Val
         35                  40

<210> 2
<211> 42
<212> PRT

<213> Homo sapiens

<400> 2

<210> 3
<211> 695
<212> PRT
<213> Homo sapiens

<400> 3
Met Leu Pro Gly Leu Ala Leu Leu Leu Leu Ala Ala Trp Thr Ala Arg
1               5                   10                  15

Ala Leu Glu Val Pro Thr Asp Gly Asn Ala Gly Leu Leu Ala Glu Pro
            20                  25                  30

Gln Ile Ala Met Phe Cys Gly Arg Leu Asn Met His Met Asn Val Gln
        35                  40                  45

Asn Gly Lys Trp Asp Ser Asp Pro Ser Gly Thr Lys Thr Cys Ile Asp
    50                  55                  60

Thr Lys Glu Gly Ile Leu Gln Tyr Cys Gln Glu Val Tyr Pro Glu Leu
65                  70                  75                  80

Gln Ile Thr Asn Val Val Glu Ala Asn Gln Pro Val Thr Ile Gln Asn
                85                  90                  95

Trp Cys Lys Arg Gly Arg Lys Gln Cys Lys Thr His Pro His Phe Val

            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205

Trp Trp Gly Gly Ala Asp Thr Asp Tyr Ala Asp Gly Ser Glu Asp Lys
    210                 215                 220

Val Val Glu Val Ala Glu Glu Glu Glu Val Ala Glu Val Glu Glu Glu
225                 230                 235                 240

Glu Ala Asp Asp Asp Glu Asp Asp Glu Asp Gly Asp Glu Val Glu Glu
                245                 250                 255

Glu Ala Glu Glu Pro Tyr Glu Glu Ala Thr Glu Arg Thr Thr Ser Ile
            260                 265                 270

Ala Thr Thr Thr Thr Thr Thr Thr Glu Ser Val Glu Glu Val Val Arg
        275                 280                 285

Val Pro Thr Thr Ala Ala Ser Thr Pro Asp Ala Val Asp Lys Tyr Leu
    290                 295                 300

Glu Thr Pro Gly Asp Glu Asn Glu His Ala His Phe Gln Lys Ala Lys
305                 310                 315                 320

Glu Arg Leu Glu Ala Lys His Arg Glu Arg Met Ser Gln Val Met Arg
                325                 330                 335

Glu Trp Glu Glu Ala Glu Arg Gln Ala Lys Asn Leu Pro Lys Ala Asp
            340                 345                 350

Lys Lys Ala Val Ile Gln His Phe Gln Glu Lys Val Glu Ser Leu Glu
        355                 360                 365

Gln Glu Ala Ala Asn Glu Arg Gln Gln Leu Val Glu Thr His Met Ala
    370                 375                 380

Arg Val Glu Ala Met Leu Asn Asp Arg Arg Arg Leu Ala Leu Glu Asn
385                 390                 395                 400

Tyr Ile Thr Ala Leu Gln Ala Val Pro Pro Arg Pro Arg His Val Phe
                405                 410                 415

Asn Met Leu Lys Lys Tyr Val Arg Ala Glu Gln Lys Asp Arg Gln His
            420                 425                 430

Thr Leu Lys His Phe Glu His Val Arg Met Val Asp Pro Lys Lys Ala
        435                 440                 445

Ala Gln Ile Arg Ser Gln Val Met Thr His Leu Arg Val Ile Tyr Glu
    450                 455                 460

Arg Met Asn Gln Ser Leu Ser Leu Leu Tyr Asn Val Pro Ala Val Ala
465                 470                 475                 480

Glu Glu Ile Gln Asp Glu Val Asp Glu Leu Leu Gln Lys Glu Gln Asn
                485                 490                 495

Tyr Ser Asp Asp Val Leu Ala Asn Met Ile Ser Glu Pro Arg Ile Ser
            500                 505                 510

Tyr Gly Asn Asp Ala Leu Met Pro Ser Leu Thr Glu Thr Lys Thr Thr
        515                 520                 525

Val Glu Leu Leu Pro Val Asn Gly Glu Phe Ser Leu Asp Asp Leu Gln
    530                 535                 540

Pro Trp His Ser Phe Gly Ala Asp Ser Val Pro Ala Asn Thr Glu Asn
545                 550                 555                 560

Glu Val Glu Pro Val Asp Ala Arg Pro Ala Ala Asp Arg Gly Leu Thr
                565                 570                 575

Thr Arg Pro Gly Ser Gly Leu Thr Asn Ile Lys Thr Glu Glu Ile Ser
            580                 585                 590

Glu Val Lys Met Asp Ala Glu Phe Arg His Asp Ser Gly Tyr Glu Val
        595                 600                 605

His His Gln Lys Leu Val Phe Phe Ala Glu Asp Val Gly Ser Asn Lys
    610                 615                 620

Gly Ala Ile Ile Gly Leu Met Val Gly Gly Val Val Ile Ala Thr Val
625                 630                 635                 640

Ile Val Ile Thr Leu Val Met Leu Lys Lys Lys Gln Tyr Thr Ser Ile
                645                 650                 655

His His Gly Val Val Glu Val Asp Ala Ala Val Thr Pro Glu Glu Arg
            660                 665                 670

His Leu Ser Lys Met Gln Gln Asn Gly Tyr Glu Asn Pro Thr Tyr Lys
        675                 680                 685

Phe Phe Glu Gln Met Gln Asn
    690                 695

<210> 4
<211> 751
<212> PRT
<213> Homo sapiens

<400> 4
Met Leu Pro Gly Leu Ala Leu Leu Leu Leu Ala Ala Trp Thr Ala Arg
1               5                   10                  15

Ala Leu Glu Val Pro Thr Asp Gly Asn Ala Gly Leu Leu Ala Glu Pro
            20                  25                  30

Gln Ile Ala Met Phe Cys Gly Arg Leu Asn Met His Met Asn Val Gln
        35                  40                  45

Asn Gly Lys Trp Asp Ser Asp Pro Ser Gly Thr Lys Thr Cys Ile Asp
    50                  55                  60

Thr Lys Glu Gly Ile Leu Gln Tyr Cys Gln Glu Val Tyr Pro Glu Leu
65                  70                  75                  80

Gln Ile Thr Asn Val Val Glu Ala Asn Gln Pro Val Thr Ile Gln Asn
                85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205

Trp Trp Gly Gly Ala Asp Thr Asp Tyr Ala Asp Gly Ser Glu Asp Lys
    210                 215                 220

Val Val Glu Val Ala Glu Glu Glu Glu Val Ala Glu Val Glu Glu Glu
225                 230                 235                 240

Glu Ala Asp Asp Asp Glu Asp Asp Glu Asp Gly Asp Glu Val Glu Glu
                245                 250                 255

Glu Ala Glu Glu Pro Tyr Glu Glu Ala Thr Glu Arg Thr Thr Ser Ile
            260                 265                 270

Ala Thr Thr Thr Thr Thr Thr Thr Glu Ser Val Glu Glu Val Val Arg
        275                 280                 285

Glu Val Cys Ser Glu Gln Ala Glu Thr Gly Pro Cys Arg Ala Met Ile
    290                 295                 300

Ser Arg Trp Tyr Phe Asp Val Thr Glu Gly Lys Cys Ala Pro Phe Phe
305                 310                 315                 320

Tyr Gly Gly Cys Gly Gly Asn Arg Asn Asn Phe Asp Thr Glu Glu Tyr
                325                 330                 335

Cys Met Ala Val Cys Gly Ser Ala Ile Pro Thr Thr Ala Ala Ser Thr
            340                 345                 350

Pro Asp Ala Val Asp Lys Tyr Leu Glu Thr Pro Gly Asp Glu Asn Glu
        355                 360                 365

His Ala His Phe Gln Lys Ala Lys Glu Arg Leu Glu Ala Lys His Arg

370                 375                 380

Glu Arg Met Ser Gln Val Met Arg Glu Trp Glu Glu Ala Glu Arg Gln
385                 390                 395                 400

Ala Lys Asn Leu Pro Lys Ala Asp Lys Lys Ala Val Ile Gln His Phe
                405                 410                 415

Gln Glu Lys Val Glu Ser Leu Glu Gln Glu Ala Ala Asn Glu Arg Gln
            420                 425                 430

Gln Leu Val Glu Thr His Met Ala Arg Val Glu Ala Met Leu Asn Asp
        435                 440                 445

Arg Arg Arg Leu Ala Leu Glu Asn Tyr Ile Thr Ala Leu Gln Ala Val
    450                 455                 460

Pro Pro Arg Pro Arg His Val Phe Asn Met Leu Lys Lys Tyr Val Arg
465                 470                 475                 480

Ala Glu Gln Lys Asp Arg Gln His Thr Leu Lys His Phe Glu His Val
                485                 490                 495

Arg Met Val Asp Pro Lys Lys Ala Ala Gln Ile Arg Ser Gln Val Met
            500                 505                 510

Thr His Leu Arg Val Ile Tyr Glu Arg Met Asn Gln Ser Leu Ser Leu
        515                 520                 525

Leu Tyr Asn Val Pro Ala Val Ala Glu Glu Ile Gln Asp Glu Val Asp
    530                 535                 540

Glu Leu Leu Gln Lys Glu Gln Asn Tyr Ser Asp Asp Val Leu Ala Asn
545                 550                 555                 560

Met Ile Ser Glu Pro Arg Ile Ser Tyr Gly Asn Asp Ala Leu Met Pro
                565                 570                 575

Ser Leu Thr Glu Thr Lys Thr Thr Val Glu Leu Leu Pro Val Asn Gly
            580                 585                 590

Glu Phe Ser Leu Asp Asp Leu Gln Pro Trp His Ser Phe Gly Ala Asp
        595                 600                 605

Ser Val Pro Ala Asn Thr Glu Asn Glu Val Glu Pro Val Asp Ala Arg
    610                 615                 620

Pro Ala Ala Asp Arg Gly Leu Thr Thr Arg Pro Gly Ser Gly Leu Thr
625                 630                 635                 640

Asn Ile Lys Thr Glu Glu Ile Ser Glu Val Lys Met Asp Ala Glu Phe
                645                 650                 655

Arg His Asp Ser Gly Tyr Glu Val His His Gln Lys Leu Val Phe Phe
            660                 665                 670

Ala Glu Asp Val Gly Ser Asn Lys Gly Ala Ile Ile Gly Leu Met Val
        675                 680                 685

Gly Gly Val Val Ile Ala Thr Val Ile Val Ile Thr Leu Val Met Leu
    690                 695                 700

Lys Lys Lys Gln Tyr Thr Ser Ile His His Gly Val Val Glu Val Asp
705                 710                 715                 720

Ala Ala Val Thr Pro Glu Glu Arg His Leu Ser Lys Met Gln Gln Asn
                725                 730                 735

Gly Tyr Glu Asn Pro Thr Tyr Lys Phe Phe Glu Gln Met Gln Asn
            740                 745                 750

<210> 5
<211> 770
<212> PRT
<213> Homo sapiens

<400> 5

<210> 6
<211> 2088

<212> DNA
<213> Homo sapiens

<220>
<221> CDS
<222> (1)..(2088)

<400> 6
atg ctg ccc ggt ttg gca ctg ctc ctg ctg gcc gcc tgg acg gct cgg 48
Met Leu Pro Gly Leu Ala Leu Leu Leu Leu Ala Ala Trp Thr Ala Arg
1               5                   10                  15

gcg ctg gag gta ccc act gat ggt aat gct ggc ctg ctg gct gaa ccc 96
Ala Leu Glu Val Pro Thr Asp Gly Asn Ala Gly Leu Leu Ala Glu Pro
            20                  25                  30

cag att gcc atg ttc tgt ggc aga ctg aac atg cac atg aat gtc cag 144
Gln Ile Ala Met Phe Cys Gly Arg Leu Asn Met His Met Asn Val Gln
        35                  40                  45

aat ggg aag tgg gat tca gat cca tca ggg acc aaa acc tgc att gat 192
Asn Gly Lys Trp Asp Ser Asp Pro Ser Gly Thr Lys Thr Cys Ile Asp
    50                  55                  60

acc aag gaa ggc atc ctg cag tat tgc.caa gaa gtc tac cct gaa ctg 240
Thr .Lys Glu Gly Ile Leu Gln Tyr Cys Gln Glu Val Tyr Pro Glu Leu
65 70 75 80

cag atc acc aat gtg gta gaa gcc aac caa cca gtg acc atc cag aac 288
Gln Ile Thr Asn Val Val Glu Ala Asn Gln Pro Val Thr Ile Gln Asn
85 90 95

tgg tgc aag cgg ggc cgc aag cag tgc aag acc cat ccc cac ttt gtg 336
Trp Cys Lys Arg Gly Arg Lys Gln Cys Lys Thr His Pro His Phe Val
100 105 110

att ccc tac cgc tgc tta gtt ggt gag ttt gta agt gat gcc ctt ctc 384
Ile Pro Tyr Arg Cys Leu Val Gly Glu Phe Val Ser Asp Ala Leu Leu

115 120 125

gtt cct gac aag tgc aaa ttc tta cac cag gag agg atg gat gtt tgc 432
Val Pro Asp Lys Cys Lys Phe Leu His Gln Glu Arg Met Asp Val Cys
130 135 140

gaa act cat ctt cac tgg cac acc gtc gcc aaa gag aca tgc agt gag 480
Glu Thr His Leu His Trp His Thr Val Ala Lys Glu Thr Cys Ser Glu
145 150 155 160

aag agt acc aac ttg cat gac tac ggc atg ttg ctg ccc tgc gga att 528
Lys Ser Thr Asn Leu His Asp Tyr Gly Met Leu Leu Pro Cys Gly Ile
165 170 175

gac aag ttc cga ggg gta gag ttt gtg tgt tgc cca ctg gct gaa gaa 576
Asp Lys Phe Arg Gly Val Glu Phe Val Cys Cys Pro Leu Ala Glu Glu
180 185 190

agt gac aat gtg gat tct gct gat gcg gag gag gat gac tcg gat gtc 624
Ser Asp Asn Val Asp Ser Ala Asp Ala Glu Glu Asp Asp Ser Asp Val
195 200 205

tgg tgg ggc gga gca gac aca gac tat gca gat ggg agt gaa gac aaa 672
Trp Trp Gly Gly Ala Asp Thr Asp Tyr Ala Asp Gly Ser Glu Asp Lys
210 215 220

gta gta gaa gta gca gag gag gaa gaa gtg gct gag gtg gaa gaa gaa 720
Val Val Glu Val Ala Glu Glu Glu Glu Val Ala Glu Val Glu Glu Glu
225 230 235 240

gaa gcc gat gat gac gag gac gat gag gat ggt gat gag gta gag gaa 768
Glu Ala Asp Asp Asp Glu Asp Asp Glu Asp Gly Asp Glu Val Glu Glu
245 250 255

gag gct gag gaa ccc tac gaa gaa gcc aca gag aga acc acc agc att 816
Glu Ala Glu Glu Pro Tyr Glu Glu Ala Thr Glu Arg Thr Thr Ser Ile
260 265 270

gcc acc acc acc acc acc acc aca gag tct gtg gaa gag gtg gtt cga 864
Ala Thr Thr Thr Thr Thr Thr Thr Glu Ser Val Glu Glu Val Val Arg
275 280 285

gtt cct aca aca gca gcc agt acc cct gat gcc gtt gac aag tat ctc 912
Val Pro Thr Thr Ala Ala Ser Thr Pro Asp Ala Val Asp Lys Tyr Leu
290 295 300

gag aca cct ggg gat gag aat gaa cat gcc cat ttc cag aaa gcc aaa 960
Glu Thr Pro Gly Asp Glu Asn Glu His Ala His Phe Gln Lys Ala Lys
305 310 315 320

gag agg ctt gag gcc aag cac cga gag aga atg tcc cag gtc atg aga 1008
Glu Arg Leu Glu Ala Lys His Arg Glu Arg Met Ser Gln Val Met Arg
325 330 335

gaa tgg gaa gag gca gaa cgt caa gca aag aac ttg cct aaa gct gat 1056
Glu Trp Glu Glu Ala Glu Arg Gln Ala Lys Asn Leu Pro Lys Ala Asp
340 345 350

aag aag gca gtt atc cag cat ttc cag gag aaa gtg gaa tct ttg gaa 1104
Lys Lys Ala Val Ile Gln His Phe Gln Glu Lys Val Glu Ser Leu Glu
355 360 365

cag gaa gca gcc aac gag aga cag cag ctg gtg gag aca cac atg gcc 1152
Gln Glu Ala Ala Asn Glu Arg Gln Gln Leu Val Glu Thr His Met Ala
370 375 380

aga gtg gaa gcc atg ctc aat gac cgc cgc cgc ctg gcc ctg gag aac 1200
Arg Val Glu Ala Met Leu Asn Asp Arg Arg Arg Leu Ala Leu Glu Asn
385 390 395 400

tac atc acc gct ctg cag gct gtt cct cct cgg cct cgt cac gtg ttc 1248
Tyr Ile Thr Ala Leu Gln Ala Val Pro Pro Arg Pro Arg His Val Phe
405 410 415

aat atg cta aag aag tat gtc cgc gca gaa cag aag gac aga cag cac 1296
Asn Met Leu Lys Lys Tyr Val Arg Ala Glu Gln Lys Asp Arg Gln His

420 425 430

acc cta aag cat ttc gag cat gtg cgc atg gtg gat ccc aag aaa gcc 1344
Thr Leu Lys His Phe Glu His Val Arg Met Val Asp Pro Lys Lys Ala
435 440 445

gct cag atc cgg tcc cag gtt atg aca cac ctc cgt gtg att tat gag 1392
Ala Gln Ile Arg Ser Gln Val Met Thr His Leu Arg Val Ile Tyr Glu
450 455 460

cgc atg aat cag tct ctc tcc ctg ctc tac aac gtg cct gca gtg gcc 1440
Arg Met Asn Gln Ser Leu Ser Leu Leu Tyr Asn Val Pro Ala Val Ala
465 470 475 480

gag gag att cag gat gaa gtt gat gag ctg ctt cag aaa gag caa aac 1488
Glu Glu Ile Gln Asp Glu Val Asp Glu Leu Leu Gln Lys Glu Gln Asn
485 490 495

tat tca gat gac gtc ttg gcc aac atg att agt gaa cca agg atc agt 1536
Tyr Ser Asp Asp Val Leu Ala Asn Met Ile Ser Glu Pro Arg Ile Ser
500 505 510

tac gga aac gat gct ctc atg cca tct ttg acc gaa acg aaa acc acc 1584
Tyr Gly Asn Asp Ala Leu Met Pro Ser Leu Thr Glu Thr Lys Thr Thr
515 520 525

gtg gag ctc ctt ccc gtg aat gga gag ttc agc ctg gac gat ctc cag 1632
Val Glu Leu Leu Pro Val Asn Gly Glu Phe Ser Leu Asp Asp Leu Gln
530 535 540

ccg tgg cat tct ttt ggg gct gac tct gtg cca gcc aac aca gaa aac 1680
Pro Trp His Ser Phe Gly Ala Asp Ser Val Pro Ala Asn Thr Glu Asn
545 550 555 560

gaa gtt gag cct gtt gat gcc cgc cct gct gcc gac cga gga ctg acc 1728
Glu Val Glu Pro Val Asp Ala Arg Pro Ala Ala Asp Arg Gly Leu Thr
565 570 575

act.cga cca ggt tct ggg ttg aca aat atc aag acg gag gag atc tct 1776
Thr Arg Pro Gly Ser Gly Leu Thr Asn Ile Lys Thr Glu Glu Ile Ser
580 585 590

gaa gtg aag atg gat gca gaa ttc cga cat gac tca gga tat gaa gtt 1824
Glu Val Lys Met Asp Ala Glu Phe Arg His Asp Ser Gly Tyr Glu Val
595 600 605

cat cat caa aaa ttg gtg ttc ttt gca gaa gat gtg ggt tca aac aaa 1872
His His Gln Lys Leu Val Phe Phe Ala Glu Asp Val Gly Ser Asn Lys
610 615 620

ggt gca atc att gga ctc atg gtg ggc ggt gtt gtc ata gcg aca gtg 1920
Gly Ala Ile Ile Gly Leu Met Val Gly Gly Val Val Ile Ala Thr Val
625 630 635 640

atc gtc atc acc ttg gtg atg ctg aag aag aaa cag tac aca tcc att 1968
Ile Val Ile Thr Leu Val Met Leu Lys Lys Lys Gln Tyr Thr Ser Ile
645 650 655

cat cat ggt gtg gtg gag gtt gac gcc gct gtc acc cca gag gag cgc 2016
His His Gly Val Val Glu Val Asp Ala Ala Val Thr Pro Glu Glu Arg
660 665 670

cac ctg tcc aag atg cag cag aac ggc tac gaa aat cca acc tac aag 2064
His Leu Ser Lys Met Gln Gln Asn Gly Tyr Glu Asn Pro Thr Tyr Lys
675 680 685

ttc ttt gag cag atg cag aac tag 2088
Phe Phe Glu Gln Met Gln Asn
690 695

<210> 7
<211> 10
<212> PRT
<213> Homo sapiens

<400> 7
Ala Ala Val Thr Pro Glu Glu Arg His Cys
1               5                   10

<210> 8
<211> 747
<212> DNA
<213> jellyfish

<220>
<221> CDS
<222> (1)..(747)

<400> 8
atg gtg agc aag ggc gag gag ctg ttc acc ggg gtg gtg ccc atc ctg     48
Met Val Ser Lys Gly Glu Glu Leu Phe Thr Gly Val Val Pro Ile Leu
1               5                   10                  15

gtc gag ctg gac ggc gac gta aac ggc cac aag ttc agc gtg tcc ggc     96
Val Glu Leu Asp Gly Asp Val Asn Gly His Lys Phe Ser Val Ser Gly
            20                  25                  30

gag ggc gag ggc gat gcc acc tac ggc aag ctg acc ctg aag ttc atc     144
Glu Gly Glu Gly Asp Ala Thr Tyr Gly Lys Leu Thr Leu Lys Phe Ile
        35                  40                  45

tgc acc acc ggc aag ctg ccc gtg ccc tgg ccc acc ctc gtg acc acc     192
Cys Thr Thr Gly Lys Leu Pro Val Pro Trp Pro Thr Leu Val Thr Thr
    50                  55                  60

ctg acc tac ggc gtg cag tgc ttc agc cgc tac ccc gac cac atg aag     240
Leu Thr Tyr Gly Val Gln Cys Phe Ser Arg Tyr Pro Asp His Met Lys
65                  70                  75                  80

cag cac gac ttc ttc aag tcc gcc atg ccc gaa ggc tac gtc cag gag     288
Gln His Asp Phe Phe Lys Ser Ala Met Pro Glu Gly Tyr Val Gln Glu
                85                  90                  95

cgc acc atc ttc ttc aag gac gac ggc aac tac aag acc cgc gcc gag 336
Arg Thr Ile Phe Phe Lys Asp Asp Gly Asn Tyr Lys Thr Arg Ala Glu
            100                 105                 110

gtg aag ttc gag ggc gac acc ctg gtg aac cgc atc gag ctg aag ggc 384
Val Lys Phe Glu Gly Asp Thr Leu Val Asn Arg Ile Glu Leu Lys Gly
        115                 120                 125

atc gac ttc aag gag gac ggc aac atc ctg ggg cac aag ctg gag tac 432
Ile Asp Phe Lys Glu Asp Gly Asn Ile Leu Gly His Lys Leu Glu Tyr
    130                 135                 140

aac tac aac agc cac aac gtc tat atc atg gcc gac aag cag aag aac 480
Asn Tyr Asn Ser His Asn Val Tyr Ile Met Ala Asp Lys Gln Lys Asn
145                 150                 155                 160

ggc atc aag gtg aac ttc aag atc cgc cac aac atc gag gac ggc agc 528
Gly Ile Lys Val Asn Phe Lys Ile Arg His Asn Ile Glu Asp Gly Ser
                165                 170                 175

gtg cag ctc gcc gac cac tac cag cag aac acc ccc atc ggc gac ggc 576
Val Gln Leu Ala Asp His Tyr Gln Gln Asn Thr Pro Ile Gly Asp Gly
            180                 185                 190

ccc gtg ctg ctg ccc gac aac cac tac ctg agc acc cag tcc gcc ctg 624
Pro Val Leu Leu Pro Asp Asn His Tyr Leu Ser Thr Gln Ser Ala Leu
        195                 200                 205

agc aaa gac ccc aac gag aag cgc gat cac atg gtc ctg ctg gag ttc 672
Ser Lys Asp Pro Asn Glu Lys Arg Asp His Met Val Leu Leu Glu Phe
    210                 215                 220

gtg acc gcc gcc ggg atc act ctc ggc atg gac gag ctg tac aag tcc 720
Val Thr Ala Ala Gly Ile Thr Leu Gly Met Asp Glu Leu Tyr Lys Ser
225                 230                 235                 240

gga ctc aga tcc acc gga tct aga taa 747

Gly Leu Arg Ser Thr Gly Ser Arg
                245

<210> 9
<211> 29
<212> DNA
<213> Artificial Sequence

<220>
<223> Description of Artificial Sequence:primer

<400> 9
tacggatccg cagaaatacg agaaactgg 29

<210> 10
<211> 30
<212> DNA
<213> Artificial Sequence

<220>
<223> Description of Artificial Sequence:primer

<400> 10
ctgaagcttt agggcggaca gaagtcggag 30

<210> 11
<211> 28
<212> DNA
<213> Artificial Sequence

<220>
<223> Description of Artificial Sequence:primer

<400> 11

tacggatccc caggcgtcca ccagtgag 28

<210> 12
<211> 29
<212> DNA
<213> Artificial Sequence

<220>
<223> Description of Artificial Sequence:primer

<400> 12
tacaagcttc atgacgcagg ctacagtgc 29

<210> 13
<211> 960
<212> DNA
<213> Homo sapiens

<220>
<223> CDK5:82-960

<400> 13
atgtcgtact accatcacca tcaccatcac gattacgata tcccaacgac cgaaaacctg 60
tattttcagg gcgccatgga tccgcagaaa tacgagaaac tggaaaagat tggggaaggc 120
acctacggaa ctgtgttcaa ggccaaaaac cgggagactc atgagatcgt ggctctgaaa 180
cgggtgaggc tggatgacga tgatgagggt gtgccgagtt ccgccctccg ggagatctgc 240
ctactcaagg agctgaagca caagaacatc gtcaggcttc atgacgtcct gcacagcgac 300
aagaagctga ctttggtttt tgaattctgt gaccaggacc tgaagaagta ttttgacagt 360
tgcaatggtg acctcgatcc tgagattgta aagtcattcc tcttccagct actaaaaggg 420
ctgggattct gtcatagccg caatgtgcta cacagggacc tgaagcccca gaacctgcta 480
ataaacagga atggggagct gaaattggct gattttggcc tggctcgagc ctttgggatt 540
cccgtccgct gttactcagc tgaggtggtc acactgtggt accgcccacc ggatgtcctc 600
tttggggcca agctgtactc cacgtccatc gacatgtggt cagccggctg catctttgca 660
gagctggcca atgctgggcg gcctcttttt cccggcaatg atgtcgatga ccagttgaag 720
aggatcttcc gactgctggg gacgcccacc gaggagcagt ggccctctat gaccaagctg 780
ccagactata agccctatcc gatgtacccg gccacaacat ccctggtgaa cgtcgtgccc 840

aaactcaatg ccacagggag ggatctgctg cagaaccttc tgaagtgtaa ccctgtccag 900
cgtatctcag cagaagaggc cctgcagcac ccctacttct ccgacttctg tccgccctaa 960

<210> 14
<211> 576
<212> DNA
<213> Homo sapiens

<220>
<223> p25

<400> 14
atgtcgtact accatcacca tcaccatcac gattacgata tcccaacgac cgaaaacctg 60
tattttcagg gcgccatgga tccccaggcg tccaccagtg agctgcttcg ctgcctgggt 120
gagtttctct gccgccggtg ctaccgcctg aagcacctgt cccccacgga ccccgtgctc 180
tggctgcgca gcgtggaccg ctcgctgctt ctgcagggct ggcaggacca gggcttcatc 240
acgccggcca acgtggtctt cctctacatg ctctgcaggg atgttatctc ctccgaggtg 300
ggctcggatc acgagctcca ggccgtcctg ctgacatgcc tgtacctctc ctactcctac 360
atgggcaacg agatctccta cccgctcaag cccttcctgg tggagagctg caaggaggcc 420
ttttgggacc gttgcctctc tgtcatcaac ctcatgagct caaagatgct gcagataaat 480
gccgacccac actacttcac acaggtcttc tccgacctga agaacgagag cggccaggag 540
gacaagaagc ggctcctcct aggcctggat cggtga 576

<210> 15
<211> 879
<212> DNA
<213> Homo sapiens

<220>
<221> CDS
<222> (1)..(879)

<400> 15
atg cag aaa tac gag aaa ctg gaa aag att ggg gaa ggc acc tac gga 48
Met Gln Lys Tyr Glu Lys Leu Glu Lys Ile Gly Glu Gly Thr Tyr Gly
1               5                   10                  15

act gtg ttc aag gcc aaa aac cgg gag act cat gag atc gtg gct ctg 96
Thr Val Phe Lys Ala Lys Asn Arg Glu Thr His Glu Ile Val Ala Leu
            20                  25                  30

aaa cgg gtg agg ctg gat gac gat gat gag ggt gtg ccg agt tcc gcc 144
Lys Arg Val Arg Leu Asp Asp Asp Asp Glu Gly Val Pro Ser Ser Ala
        35                  40                  45

ctc cgg gag atc tgc cta ctc aag gag ctg aag cac aag aac atc gtc 192
Leu Arg Glu Ile Cys Leu Leu Lys Glu Leu Lys His Lys Asn Ile Val
    50                  55                  60

agg ctt cat gac gtc ctg cac agc gac aag aag ctg act ttg gtt ttt 240
Arg Leu His Asp Val Leu His Ser Asp Lys Lys Leu Thr Leu Val Phe
65                  70                  75                  80

gaa ttc tgt gac cag gac ctg aag aag tat ttt gac agt tgc aat ggt 288
Glu Phe Cys Asp Gln Asp Leu Lys Lys Tyr Phe Asp Ser Cys Asn Gly
                85                  90                  95

gac ctc gat cct gag att gta aag tca ttc ctc ttc cag cta cta aaa 336
Asp Leu Asp Pro Glu Ile Val Lys Ser Phe Leu Phe Gln Leu Leu Lys
            100                 105                 110

ggg ctg gga ttc tgt cat agc cgc aat gtg cta cac agg gac ctg aag 384
Gly Leu Gly Phe Cys His Ser Arg Asn Val Leu His Arg Asp Leu Lys
        115                 120                 125

ccc cag aac ctg cta ata aac agg aat ggg gag ctg aaa ttg gct aat 432
Pro Gln Asn Leu Leu Ile Asn Arg Asn Gly Glu Leu Lys Leu Ala Asn
    130                 135                 140

ttt ggc ctg gct cga gcc ttt ggg att ccc gtc cgc tgt tac tca gct 480
Phe Gly Leu Ala Arg Ala Phe Gly Ile Pro Val Arg Cys Tyr Ser Ala
145                 150                 155                 160

gag gtg gtc aca ctg tgg tac cgc cca ccg gat gtc ctc ttt ggg gcc 528

Glu Val Val Thr Leu Trp Tyr Arg Pro Pro Asp Val Leu Phe Gly Ala
                165                 170                 175

aag ctg tac tcc acg tcc atc gac atg tgg tca gcc ggc tgc atc ttt 576
Lys Leu Tyr Ser Thr Ser Ile Asp Met Trp Ser Ala Gly Cys Ile Phe
            180                 185                 190

gca gag ctg gcc aat gct ggg cgg cct ctt ttt ccc ggc aat gat gtc 624
Ala Glu Leu Ala Asn Ala Gly Arg Pro Leu Phe Pro Gly Asn Asp Val
        195                 200                 205

gat gac cag ttg aag agg atc ttc cga ctg ctg ggg acg ccc acc gag 672
Asp Asp Gln Leu Lys Arg Ile Phe Arg Leu Leu Gly Thr Pro Thr Glu
    210                 215                 220

gag cag tgg ccc tct atg acc aag ctg cca gac tat aag ccc tat ccg 720
Glu Gln Trp Pro Ser Met Thr Lys Leu Pro Asp Tyr Lys Pro Tyr Pro
225                 230                 235                 240

atg tac ccg gcc aca aca tcc ctg gtg aac gtc gtg ccc aaa ctc aat 768
Met Tyr Pro Ala Thr Thr Ser Leu Val Asn Val Val Pro Lys Leu Asn
                245                 250                 255

gcc aca ggg agg gat ctg ctg cag aac ctt ctg aag tgt aac cct gtc 816
Ala Thr Gly Arg Asp Leu Leu Gln Asn Leu Leu Lys Cys Asn Pro Val
            260                 265                 270

cag cgt atc tca gca gaa gag gcc ctg cag cac ccc tac ttc tcc gac 864
Gln Arg Ile Ser Ala Glu Glu Ala Leu Gln His Pro Tyr Phe Ser Asp
        275                 280                 285

ttc tgt ccg ccc tag 879
Phe Cys Pro Pro
    290

<210> 16
<211> 30

<212> DNA
<213> Artificial Sequence

<220>
<223> Description of Artificial Sequence:primer

<400> 16
ctgaagcttc gcagaaatac gagaaactgg                                        30

<210> 17
<211> 30
<212> DNA
<213> Artificial Sequence

<220>
<223> Description of Artificial Sequence:primer

<400> 17
gatctcgagt agggcggaca gaagtcggag                                        30

<210> 18
<211> 33
<212> DNA
<213> Artificial Sequence

<220>
<223> Description of Artificial Sequence:primer

<400> 18
ggagctgaaa ttggctaatt ttggcctggc tcg                                    33

<210> 19
<211> 33
<212> DNA
<213> Artificial Sequence

<220>
<223> Description of Artificial Sequence:primer

<400> 19
cgagccaggc caaaattagc caatttcagc tcc 33

<210> 20
<211> 31
<212> DNA
<213> Artificial Sequence

<220>
<223> Description of Artificial Sequence:primer

<400> 20
ctgaagctta tgcagaaata cgagaaactg g 31

<210> 21
<211> 30
<212> DNA
<213> Artificial Sequence

<220>
<223> Description of Artificial Sequence:primer

<400> 21
gatgtcgact agggcggaca gaagtcggag 30